# FASCICULE DE BREVET EUROPEEN

(11) **EP 3 294 111 B1**
(45) Date de publication et mention de la délivrance du brevet: **07.08.2019**
(21) Numéro de dépôt: 16729959.3
(22) Date de dépôt: 11.05.2016
(51) Int. Cl.: A61B 1/04, A61B 1/06, A61B 1/07, A61B 1/00, A61B 1/045

(54) **DISPOSITIF ET PROCÉDÉ D'OBSERVATION D'UN OBJET, AVEC PRISE EN COMPTE DE LA DISTANCE ENTRE LE DISPOSITIF ET L'OBJET**
VORRICHTUNG UND VERFAHREN ZUR BEOBACHTUNG EINES OBJEKTS UNTER BERÜCKSICHTIGUNG DES ABSTANDS ZWISCHEN DER VORRICHTUNG UND DEM OBJEKT
DEVICE AND METHOD FOR OBSERVING AN OBJECT, TAKING INTO CONSIDERATION THE DISTANCE BETWEEN THE DEVICE AND THE OBJECT

(30) Priorité: 12.05.2015 FR 1554259
(43) Date de publication de la demande: 21.03.2018
(73) Titulaire: Commissariat à l'énergie atomique et aux énergies alternatives, 75015 Paris (FR); Fluoptics, 38040 Grenoble (FR)
(72) Inventeur: RIZO, Philippe, 38700 La Tronche (FR)
(74) Mandataire: GIE Innovation Competence Group
(86) Numéro de dépôt international: PCT/FR2016/051115
(87) Numéro de publication internationale: WO 2016/181077

(56) Documents cités:
- EP-A1- 1 167 951
- EP-A2- 2 020 202
- JP-A- 2011 227 132
- US-A1- 2010 157 039

## Description

### DOMAINE TECHNIQUE

Le domaine de l'invention est l'imagerie réalisée sur des objets, en particulier à l'aide de guides de lumière de type endoscope ou laparoscope. Les objets peuvent notamment être des tissus corporels. L'imagerie peut être l'imagerie de fluorescence pour le diagnostic et le suivi de l'évolution de pathologies ou de traitements.

### ART ANTERIEUR

L'imagerie de fluorescence est une technique permettant de localiser des marqueurs fluorescents dans un corps humain ou animal. Une application est la localisation de marqueurs fluorescents, ou fluorophores, ces derniers ciblant des cellules d'intérêt, par exemple des cellules cancéreuses. Le protocole consiste à injecter ces marqueurs dans l'organisme avant une intervention chirurgicale, de telle sorte qu'au cours de l'intervention, le praticien soit en mesure de visualiser les cellules cancéreuses à l'aide d'une l'image de fluorescence. Parce qu'elle permet d'acquérir une image indiquant la localisation des différentes zones cancéreuses, l'imagerie de fluorescence peropératoire permet d'obtenir une information jusqu'alors inaccessible au praticien, et constitue un complément utile, voire une alternative, à l'utilisation de traceurs radioactifs. Une autre application est l'assistance à des interventions en chirurgie cardiovasculaire, en chirurgie du système lymphatique, ou en chirurgie hépatique, où l'imagerie de fluorescence permet le contrôle visuel du drainage, de la perfusion ou de la vascularisation.

Le principe de l'imagerie de fluorescence est d'illuminer un champ d'observation à l'aide d'une source de lumière dans une bande spectrale d'excitation des fluorophores. Sous l'effet de cette illumination, les fluorophores émettent un rayonnement de fluorescence, dans une bande spectrale de fluorescence. Ce rayonnement peut être capté par une caméra de fluorescence, de manière à former une image de fluorescence sur laquelle apparaissent les différentes zones fluorescentes. Il est alors possible d'acquérir une image visible du champ observé, et de superposer l'image de fluorescence sur cette image visible.

Une modalité d'application de l'imagerie de fluorescence est la voie endoscopique ou laparoscopique, permettant d'acquérir des images de fluorescence d'un tissu biologique à l'intérieur d'un organisme, par une voie d'accès minimalement invasive. Le document US20130184591 décrit par exemple un laparoscope comprenant une extrémité distale, destinée à être introduite dans un organisme, à proximité du tissu examiné, et une extrémité proximale, destinée à être maintenue à l'extérieur de l'organisme.

L'extrémité proximale est raccordée à une source de lumière, produisant un faisceau d'excitation permettant l'émission d'une lumière de fluorescence par des fluorophores potentiellement présents dans le tissu, les marqueurs étant préalablement introduits dans l'organisme à examiner. Elle est également raccordée à une source de lumière visible. Au niveau de son extrémité proximale, le laparoscope comprend par ailleurs un capteur d'image visible et un capteur d'image de fluorescence. Des moyens optiques de séparation spectrale permettent de diriger la lumière visible réfléchie par le tissu biologique ainsi que la lumière de fluorescence respectivement vers le capteur d'image visible et le capteur d'image de fluorescence.

La transmission de la lumière de fluorescence et de la lumière visible du tissu examiné vers l'extrémité proximale nécessite un système optique de collection, placée au niveau de l'extrémité distale, pour collecter les signaux optiques dans un certain champ d'observation. Elle nécessite par ailleurs un système optique de transmission, ou relais optique, pour transmettre les lumières de fluorescence et visible vers leurs capteurs d'images respectifs, disposés au niveau de l'extrémité proximale.

Du fait de la faible ouverture de ces systèmes optiques, la quantité de signal de fluorescence parvenant à l'extrémité proximale est faible. Elle peut être augmentée, moyennant une augmentation de l'intensité du faisceau d'excitation. Se pose alors le problème des risques de dessèchement ou de brûlure du tissu examiné, du fait de l'augmentation du signal lumineux d'excitation. En particulier, lorsque le faisceau d'excitation est produit par une source laser, il convient de respecter des exigences réglementaires, par exemple la norme internationale IEC 60825-1, relative à la sécurité des appareils à laser. Le dispositif décrit dans ce document permet un contrôle de l'exposition du tissu en analysant les signaux détectés par les capteurs d'image logés dans l'extrémité proximale du laparoscope.

Le document EP1167951 décrit un endoscope pour l'analyse de la fluorescence d'un échantillon. Ce document décrit comment, en se basant sur une image visible de l'échantillon ou sur une image de fluorescence de ce dernier, et plus précisément sur le nombre de pixels dont l'intensité franchit un certain seuil, on peut déterminer qu'une certaine distance critique est franchie entre une extrémité de l'endoscope et la surface de l'échantillon. L'intensité d'un faisceau d'excitation peut être ajustée en conséquence. Cependant, la détection du franchissement de la distance critique dépend des propriétés optiques de réflexion et/ou de rétrodiffusion de l'échantillon. Le document US20100157039 décrit un endoscope pour l'analyse de la fluorescence d'un échantillon, permettant une mesure d'une distance entre une extrémité de l'endoscope et une surface de l'échantillon. La distance est déterminée sur la base d'une image infrarouge de l'échantillon, la surface de ce dernier subissant un balayage par un faisceau lumineux infrarouge, le balayage prenant la forme d'une spirale.

Le document EP 2020202 décrit un endoscope pour l'analyse de la fluorescence d'un échantillon. Sur la base d'une évaluation du franchissement d'une distance critique entre une extrémité de l'endoscope et une surface de l'échantillon analysée, une source de lumière auxiliaire peut être modulée, de façon à prévenir une inhomogénéité d'un éclairement de l'échantillon. Le franchissement de la distance critique est évalué en se basant sur une luminance d'une image de fluorescence acquise par l'endoscope.

Mais un contrôle de l'exposition de tissus, ou la détermination d'une distance, par un capteur d'image manque de précision, et peut en particulier dépendre trop fortement des propriétés optiques de réflexion ou de rétrodiffusion de l'échantillon. Les inventeurs ont conçu un dispositif et un procédé d'observation d'un objet, notamment un tissu corporel, et en particulier par voie endoscopique ou laparoscopique, permettant d'acquérir une image de fluorescence, tout en s'assurant que l'illumination du tissu examiné soit conforme aux exigences en vigueur à l'égard de l'intégrité de ces tissus, garantissant une bonne sécurité d'utilisation.

### EXPOSE DE L'INVENTION

Un objet de l'invention est un dispositif d'observation d'un objet comportant :
- une source de lumière d'excitation apte à produire un faisceau d'excitation, dans une bande spectrale d'excitation, se propageant vers ledit objet,
- un capteur d'image d'émission, apte à collecter une lumière d'émission émise par ledit objet, sous l'effet dudit faisceau d'excitation, dans une bande spectrale d'émission, le capteur d'image d'émission étant apte à acquérir une image d'émission à partir de la lumière d'émission collectée,
- un capteur de distance comportant une source de lumière de télémétrie, apte à produire un faisceau de télémétrie, dans une bande spectrale de télémétrie, se propageant vers l'objet,
- le capteur de distance comportant également un capteur de lumière de télémétrie, apte à détecter une lumière de télémétrie réfléchie par l'objet,
le dispositif étant caractérisé en ce qu'il comporte :
- un élément projecteur, configuré pour projeter ledit faisceau d'excitation et ledit faisceau de télémétrie, vers l'objet, selon un angle solide de projection.
- le capteur de distance étant apte à mesurer une distance entre ledit élément projecteur et ledit objet,
- un modulateur, configuré pour moduler une intensité du faisceau d'excitation, en fonction de ladite distance mesurée par le capteur de distance.

Le dispositif peut notamment comporter un guide de lumière, s'étendant entre une extrémité proximale et une extrémité distale, le guide de lumière étant apte à transmettre :
- le faisceau d'excitation et le faisceau de télémétrie de ladite extrémité proximale vers ladite extrémité distale,
- la lumière d'émission et ladite lumière de télémétrie réfléchie par l'objet de ladite extrémité distale vers ladite extrémité proximale,
l'extrémité distale dudit guide de lumière comportant ledit élément projecteur.

En particulier, l'extrémité distale est destinée à être introduite dans le corps d'un humain ou d'un animal, de façon à pouvoir être disposée face à l'objet à examiner. L'objet à examiner est notamment un tissu biologique, en particulier un tissu corporel.

Le guide de lumière peut être un laparoscope ou un endoscope.

Le guide de lumière peut comporter, au niveau de son extrémité distale, un système optique apte à collecter la lumière d'émission et la lumière de télémétrie provenant de l'objet, de façon à les diriger vers l'extrémité proximale du guide de lumière. Ce système optique peut notamment comporter un objectif ou une lentille. Ce système optique définit un angle solide d'observation, dont l'intersection avec l'objet forme le champ observé.

Ce système optique peut également être apte à diriger ledit faisceau d'excitation et ledit faisceau de télémétrie vers ledit objet, ledit système optique formant alors l'élément projecteur. Le guide de lumière peut comporter au moins une fibre optique de transmission, s'étendant entre l'extrémité proximale et l'extrémité distale du guide de lumière, de façon à guider le faisceau d'excitation et le faisceau de télémétrie entre ladite extrémité proximale et ladite extrémité distale, l'extrémité de chaque fibre optique de transmission au niveau de ladite extrémité distale formant l'élément projecteur.

De préférence, l'élément projecteur dirige le faisceau d'excitation et le faisceau de télémétrie selon le même angle solide de projection.

Le capteur de distance est apte à mesurer une durée entre l'émission du faisceau de télémétrie par la source de lumière de télémétrie, et la détection, par le capteur de lumière de télémétrie, de la lumière de télémétrie réfléchie par l'objet.

La projection du faisceau d'excitation et du faisceau de télémétrie définit un champ éclairé sur l'objet. De préférence, le champ éclairé est confondu avec ou inscrit dans le champ observé. Le guide de lumière peut comporter un séparateur spectral, apte à diriger :
- la lumière d'émission vers le capteur d'image d'émission,
- la lumière de télémétrie réfléchie par l'objet vers le capteur de lumière de télémétrie.

Le séparateur spectral, le capteur d'image d'émission et le capteur de lumière de télémétrie peuvent être logés au niveau de l'extrémité proximale du guide de lumière, par exemple dans un module de détection situé à cette extrémité proximale.

Le dispositif peut comporter :
- une source de lumière visible, apte à produire un faisceau lumineux visible vers ledit objet dans une bande spectrale visible,
- un capteur d'image visible, apte à collecter une lumière visible réfléchie par l'objet sous l'effet d'une illumination par ledit faisceau lumineux visible, le capteur d'image visible étant apte à acquérir une image visible à partir de la lumière visible collectée,

le guide de lumière étant alors apte à transmettre :
- ledit faisceau lumineux visible de l'extrémité proximale vers l'extrémité distale,
- et ladite lumière visible réfléchie par l'objet de l'extrémité distale vers l'extrémité proximale.

Le capteur d'image visible peut être logé au niveau de l'extrémité proximale du guide de lumière, par exemple dans le module de détection intégré à cette extrémité proximale.

La source de lumière de télémétrie peut être confondue avec la source de lumière d'excitation. Dans ce cas, la mesure de distance est réalisée en utilisant la propagation de la lumière d'excitation de la source de lumière d'excitation vers l'objet ainsi que la propagation de la lumière d'excitation réfléchie par l'objet entre ce dernier et le capteur de lumière de télémétrie. La bande spectrale de télémétrie peut être différente de la bande spectrale d'excitation et de la bande spectrale d'émission, voire de la bande spectrale visible.

Le capteur de distance peut comporter :
- un répartiteur, apte à renvoyer une partie dudit faisceau de télémétrie, émis par la source de télémétrie, vers un photodétecteur de déclenchement, ce dernier étant apte à détecter ladite partie du faisceau de télémétrie renvoyée par ledit répartiteur,
- un processeur de télémétrie, apte à déterminer une distance de parcours du faisceau de télémétrie entre la source de lumière de télémétrie et le capteur de télémétrie, en fonction d'un instant de déclenchement, auquel le photodétecteur de déclenchement détecte ledit faisceau de télémétrie et d'un instant d'arrêt, auquel ledit capteur de lumière de télémétrie détecte ladite lumière de télémétrie réfléchie par l'objet,
- le processeur de télémétrie étant apte à déterminer ladite distance entre l'élément de projecteur et l'objet à partir de la distance de parcours du faisceau de télémétrie.

Le photodétecteur de déclenchement et le processeur de télémétrie peuvent être inclus dans le guide de lumière.

Un autre objet de l'invention est un procédé d'observation d'un objet comportant les étapes suivantes :
- illumination d'un objet par un faisceau lumineux de télémétrie, émis par une source de lumière de télémétrie, dans une bande spectrale de télémétrie, le faisceau d'illumination étant projeté sur l'objet par un élément projecteur définissant un angle solide de projection,
- détection d'une lumière de télémétrie réfléchie par l'objet, dans ladite bande spectrale de télémétrie,
- en fonction de ladite lumière de télémétrie détectée, mesure d'une distance entre l'élément projecteur et l'objet, notamment en fonction d'une durée entre l'émission du faisceau de télémétrie et la détection de la lumière de télémétrie réfléchie par l'objet
- illumination dudit objet à l'aide d'un faisceau d'excitation émis par une source de lumière d'excitation, dans une bande spectrale d'excitation, ledit faisceau d'excitation étant projeté sur l'objet par ledit élément projecteur, selon ledit angle solide de projection,
- détection d'une lumière d'émission par un capteur d'image d'émission, ladite lumière d'émission étant émise par l'objet sous l'effet de ladite illumination par le faisceau d'excitation,
- acquisition d'une image d'émission par ledit capteur d'image d'émission à partir de ladite lumière d'émission détectée,
le procédé étant caractérisé en ce qu'il comporte également :
- la modulation d'une intensité du faisceau d'excitation, en fonction de la distance mesurée.

La modulation de l'intensité du faisceau d'excitation peut être réalisée en modulant un signal de commande de la source de lumière d'excitation ou en intercalant un atténuateur entre la source de lumière d'excitation et l'objet.

Selon un mode de réalisation,
- le faisceau d'excitation et le faisceau lumineux de télémétrie sont transmis jusqu'à l'objet par un guide de lumière, dont une extrémité distale, comprenant l'élément projecteur, est placée face à l'objet,
- le guide de lumière assurant également la transmission de ladite lumière d'émission émise par l'objet et de ladite lumière de télémétrie réfléchie par l'objet respectivement vers le capteur d'image d'émission et le capteur de lumière de télémétrie.

Le capteur d'image d'émission et le capteur de lumière de télémétrie peuvent notamment être disposés au niveau de l'extrémité proximale du guide de lumière, par exemple dans un module de détection intégré dans cette extrémité proximale.

La lumière d'émission émise par l'objet et la lumière de télémétrie réfléchie par l'objet peuvent être collectées par un système optique situé à l'extrémité distale du guide de lumière, avant d'être renvoyées respectivement vers le capteur d'image d'émission et le capteur de lumière de télémétrie. Le système optique définit un champ observé sur l'objet, tel que précédemment défini.

Ce système optique, pouvant notamment comporter un objectif ou une lentille, définit un champ observé sur l'objet, correspondant à une surface de l'objet de laquelle proviennent la lumière d'émission et la lumière de télémétrie collectées par le système optique.

Selon un mode de réalisation, le faisceau d'excitation et le faisceau de télémétrie sont projetés sur l'objet par ledit système optique, ce dernier formant alors ledit élément projecteur.

Selon un mode de réalisation, le faisceau d'excitation et le faisceau de télémétrie sont projetés sur l'objet par au moins une fibre optique, s'étendant entre l'extrémité proximale et l'extrémité distale du guide de lumière, l'extrémité de chaque fibre optique au niveau de ladite extrémité distale formant l'élément projecteur.

Selon un mode de réalisation, la lumière d'émission est une lumière de fluorescence, dans une bande spectrale d'émission, ou bande spectrale de fluorescence, différente de la bande spectrale d'excitation.

Selon un mode de réalisation, la lumière d'émission correspond à une réflexion ou du faisceau d'excitation sur l'objet et/ou à une rétrodiffusion du faisceau d'excitation dans l'objet. La bande spectrale d'émission correspond donc à la bande spectrale d'excitation.

Selon un mode de réalisation, le procédé comporte également :
- l'illumination de l'objet par un faisceau lumineux visible produit par une source de lumière visible, dans une bande spectrale visible, notamment à travers le guide de lumière,
- l'acquisition d'une image visible de l'objet à l'aide d'un capteur d'image visible, ce dernier détectant une lumière visible réfléchie par l'objet, notamment à travers le guide de lumière.

Selon un mode de réalisation, la source de lumière de télémétrie est confondue avec la source de lumière d'excitation, la bande spectrale de télémétrie correspondant alors à la bande spectrale d'excitation.

Selon un mode de réalisation, la bande spectrale de télémétrie est différente de la bande spectrale d'excitation et de la bande spectrale d'émission, voire de la bande spectrale visible. Un autre objet de l'invention est un dispositif comportant :
- un capteur de distance comportant une source de lumière de télémétrie, apte à émettre un faisceau de télémétrie, dans une bande spectrale de télémétrie, se propageant vers l'objet,
- le capteur de distance comportant également un capteur de lumière de télémétrie, apte à détecter une lumière de télémétrie réfléchie par l'objet,
le dispositif étant caractérisé en ce qu'il comporte :
- un guide de lumière, s'étendant entre une extrémité proximale et une extrémité distale, le guide de lumière étant apte à transmettre le faisceau de télémétrie de ladite extrémité proximale vers ladite extrémité distale, ainsi que ladite lumière de télémétrie réfléchie par l'objet de l'extrémité distale vers ladite extrémité proximale,
- l'extrémité distale dudit guide de lumière comportant un élément projecteur pour projeter ledit faisceau de télémétrie, vers l'objet, selon un angle solide de projection,
- le capteur de distance étant apte à mesurer une distance entre ledit élément projecteur et ledit objet.

En particulier, l'extrémité distale est destinée à être introduite dans le corps d'un humain ou d'un animal, de façon à pouvoir être notamment disposée face à l'objet à examiner. L'objet à examiner est notamment un tissu biologique, en particulier un tissu corporel. Le guide de lumière peut notamment être un laparoscope ou un endoscope.

Le guide de lumière peut comporter, au niveau de son extrémité distale, un système optique apte à collecter la lumière de télémétrie provenant de l'objet, de façon à la diriger vers l'extrémité proximale du guide de lumière. Ce système optique peut notamment comporter un objectif ou une lentille. Ce système optique définit un angle solide d'observation, dont l'intersection avec l'objet forme le champ observé.

De préférence, la surface de l'objet est divisée en éléments de surface. Le capteur de distance est alors apte à mesurer une distance entre ledit élément projecteur et chaque élément de surface. Le capteur de distance peut notamment comprendre un photodétecteur matriciel, comportant une pluralité de pixels, et est apte à déterminer une distance entre chaque pixel et chaque élément de surface auquel ledit pixel est optiquement couplé.

Selon un mode de réalisation, le dispositif comprend un processeur, relié au capteur de distance, et apte à établir une cartographie tridimensionnelle du champ observé.

Le capteur de lumière de télémétrie peut notamment être disposé au niveau de l'extrémité proximale du guide de lumière, par exemple dans un module de détection inclus dans cette extrémité proximale.

Le dispositif peut comporter :
- une source de lumière d'excitation apte à produire un faisceau d'excitation, dans une bande spectrale d'excitation se propageant vers un objet,
- un capteur d'image d'émission, apte à collecter une lumière d'émission émise par ledit objet, sous l'effet dudit faisceau d'excitation, dans une bande spectrale d'émission, le capteur d'image d'émission étant apte à acquérir une image d'émission à partir de la lumière d'émission collectée,
- le guide de lumière étant apte à transmettre ledit faisceau d'excitation vers l'objet et à transmettre ladite lumière d'émission réfléchie par l'objet vers ledit capteur d'image d'émission. Ce dernier peut notamment être disposé dans le guide de lumière, en particulier au niveau de son extrémité proximale.

Un autre objet de l'invention est un procédé comportant les étapes suivantes :
- illumination d'un objet par un faisceau lumineux de télémétrie, produit par une source de lumière de télémétrie, dans une bande spectrale de télémétrie, le faisceau de télémétrie étant projeté sur l'objet par un élément projecteur définissant un angle solide de projection,
- détection d'une lumière de télémétrie réfléchie par l'objet, dans ladite bande spectrale de télémétrie, par un capteur de lumière de télémétrie ;
- en fonction de ladite lumière de télémétrie détectée, mesure d'une distance entre l'élément projecteur et l'objet,
le procédé étant caractérisé en ce que :
- le faisceau de télémétrie est transmis jusqu'à l'objet par un guide de lumière, dont une extrémité distale, comprenant l'élément projecteur, est placée face à l'objet,
- le guide de lumière assurant également la transmission de ladite lumière de télémétrie réfléchie par l'objet vers le capteur de lumière de télémétrie.

La lumière de télémétrie réfléchie par l'objet peut être collectée par un système optique situé à l'extrémité distale du guide de lumière, avant d'être renvoyée vers le capteur de lumière de télémétrie. Le système optique définit un champ observé sur l'objet, tel que précédemment défini.

Selon un mode de réalisation, la surface de l'objet est divisée en éléments de surface. Le procédé comporte alors une étape de mesure de la distance entre ledit élément projecteur et chaque élément de surface. Selon ce mode de réalisation, le capteur de distance peut notamment comprendre un photodétecteur matriciel. Le photodétecteur matriciel comporte une pluralité de pixels, de façon à déterminer, une distance séparant chaque pixel de l'élément de surface auquel ledit pixel est optiquement couplé.

Selon ce mode de réalisation, le procédé peut comporter la réalisation une cartographie tridimensionnelle du champ observé, en fonction de la distance mesurée correspondant à chaque élément de surface.

Le procédé peut comporter les étapes suivantes :
- illumination dudit objet à l'aide d'un faisceau d'excitation émis par une source de lumière d'excitation, dans une bande spectrale d'excitation, ledit faisceau d'excitation étant projeté sur l'objet par ledit élément projecteur, selon ledit angle solide de projection,
- détection d'une lumière d'émission par un capteur d'image d'émission, ladite lumière d'émission étant émise par l'objet sous l'effet de ladite illumination par le faisceau d'excitation,
- acquisition d'une image d'émission par ledit capteur d'image d'émission à partir de ladite lumière d'émission détectée.

Selon cette variante, le faisceau d'excitation peut être transmis jusqu'à l'objet par ledit guide de lumière, ce dernier assurant également la transmission de ladite lumière d'émission émise par l'objet vers le capteur d'image d'émission.

### FIGURES

La figure 1A représente un premier mode de réalisation d'un dispositif selon l'invention.
Les figures 1B et 1C représentent un détail de l'extrémité distale du guide de lumière selon deux variantes différentes.
La figure 1D représente un détail de l'extrémité distale du guide de lumière et de l'objet.
La figure 2 représente un détail de la structure du capteur de distance.
La figure 3 représente un détail de l'extrémité proximale du guide de lumière.
La figure 4 représente les étapes d'un procédé selon l'invention.
La figure 5 représente un deuxième mode de réalisation d'un dispositif selon l'invention.
La figure 6 représente une variante du premier ou du deuxième mode de réalisation d'un dispositif selon l'invention.
La figure 7 représente une autre variante du premier ou du deuxième mode de réalisation d'un dispositif selon l'invention.
La figure 8 représente une autre variante du premier ou du deuxième mode de réalisation d'un dispositif selon l'invention.
La figure 9 représente un montage expérimental.
La figure 10 représente les résultats d'un essai réalisé en mettant en oeuvre le montage expérimental décrit dans la figure 9.
La figure 11 représente un troisième mode de réalisation d'un dispositif selon l'invention.

### EXPOSE DE MODES DE REALISATION PARTICULIERS

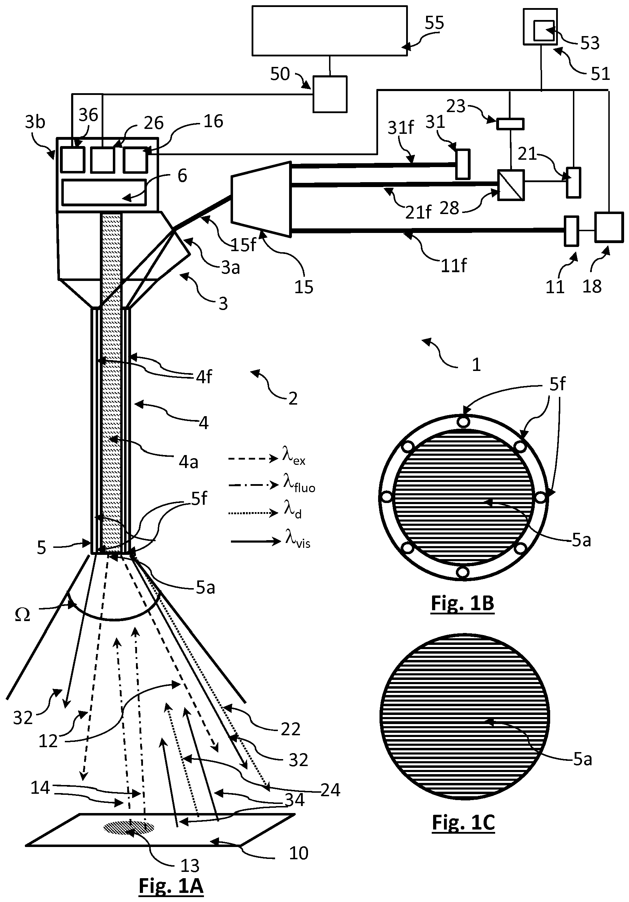

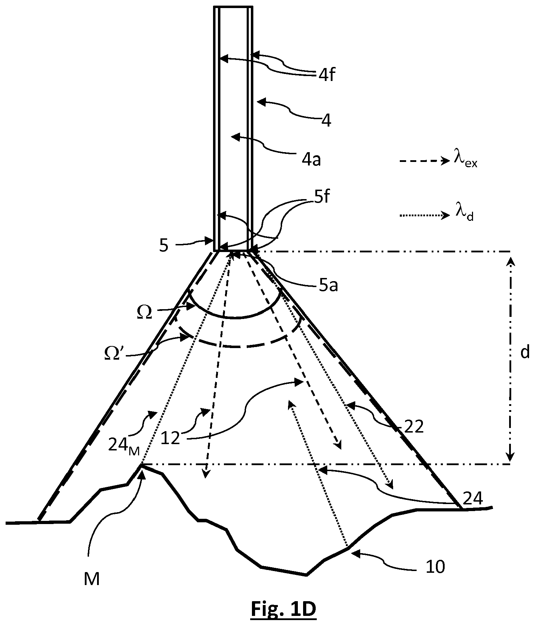

La figure 1 représente un dispositif 1 selon un premier mode de réalisation. Le dispositif comporte une source de lumière d'excitation 11, apte à émettre un faisceau lumineux d'excitation 12, dans une bande spectrale d'excitation λₑₓ, de façon à illuminer un objet 10. L'objet 10 est par exemple un tissu biologique, exposé au dispositif 1 au cours d'une intervention endoscopique ou laparoscopique.

Le terme bande spectrale désigne l'ensemble des longueurs d'onde comprise dans un intervalle déterminé, entre une longueur d'onde minimale et une longueur d'onde maximale.

La source de lumière d'excitation 11 peut être continue, modulée en amplitude, ou impulsionnelle.

La source de lumière d'excitation 11 est dans cet exemple une diode laser pulsée, émettant à une longueur d'onde λₑₓ= 750 nm. Il peut également s'agir d'une diode électroluminescente.

La source de lumière peut comprendre un filtre d'excitation, apte à bloquer les longueurs d'onde en dehors de la bande spectrale d'excitation λₑₓ.

Le dispositif comporte un guide de lumière 2, comportant une extrémité proximale 3 et une extrémité distale 5. Le guide de lumière permet la transmission d'une lumière entre l'extrémité proximale 3 et l'extrémité distale 5 et réciproquement.

Ce guide de lumière est, dans cet exemple, un laparoscope. Il peut également s'agir d'un endoscope. L'extrémité proximale 3 comprend une fenêtre d'entrée 3a, apte à recevoir des faisceaux lumineux destinés à être transmis jusqu'à l'extrémité distale 5. L'extrémité proximale comprend également un module de détection 3b, permettant la détection de rayons lumineux collectés par l'extrémité distale 5.

Dans cet exemple, le faisceau lumineux d'excitation émis par la source de lumière 11 est transmis par une fibre optique d'excitation 11f (ou une pluralité de fibres optiques d'excitation), assurant le transport du faisceau d'excitation entre la source de lumière d'excitation 11 et un coupleur optique 15. Une fibre optique de couplage 15f, ou une pluralité de fibres optiques de couplage 15f, assure le transport du faisceau d'excitation 12 entre le coupleur optique 15 et la fenêtre optique d'entrée 3a. Cette dernière comporte un connecteur optique permettant la transmission du faisceau d'excitation dans le guide de lumière 2.

Le guide de lumière 2 comporte un tronçon central 4, s'étendant entre l'extrémité proximale 3 et l'extrémité distale 5, de façon à transmettre une lumière entre ces deux extrémités. Sur l'exemple représenté, le tronçon central 4 comporte une pluralité de fibres optiques de transmission 4f destinées à la transmission du faisceau lumineux d'excitation 12 entre l'extrémité proximale 3 et l'extrémité distale 5. Le tronçon central 4 comporte également un élément optique de relais 4a, connu en soi, comprenant par exemple des lentilles relais, et apte à transférer une lumière, et notamment une image, entre l'extrémité distale 5 et l'extrémité proximale 3, et réciproquement. Les fibres optiques 4f sont par exemple réparties à la périphérie du tronçon relais, l'élément optique de relais 4a étant disposé dans la partie centrale du tronçon central 4.Généralement, le guide de lumière 2 comporte plusieurs dizaines voire centaines de fibres optiques de transmission 4f.

Selon une variante, le tronçon central est souple et comporte un faisceau de fibres optiques. Le diamètre du tronçon central est généralement inférieur à 1 ou 2 cm.

La longueur du tronçon central est par exemple comprise entre 10 et 30 cm lorsque le guide de lumière est rigide, par exemple lorsqu'il s'agit d'un laparoscope. Dans le cas d'un endoscope, la longueur du guide peut dépasser 1 mètre, voire plusieurs mètres.

L'extrémité distale 5 est destinée à être introduite dans le corps d'un animal ou d'un humain. Elle comporte un élément projecteur destiné à projeter le faisceau d'excitation 12 sur l'objet 10. La lumière est émise, depuis l'élément projecteur, selon un cône d'émission définissant un angle solide de projection Ω. L'intersection du cône d'émission avec l'objet 10 correspond au champ éclairé S_{Ω}.

Par élément projecteur, on entend un élément optique permettant la projection d'un faisceau de lumière sur l'objet, selon un cône d'émission. Il peut notamment s'agir de l'extrémité d'une ou plusieurs fibres optiques ou d'un système optique de type lentille ou objectif.

Dans l'exemple représenté sur la figure 1A, l'extrémité distale 5f de chaque fibre optique de transmission 4f débouche au niveau de l'extrémité distale 5 du guide. L'élément projecteur est alors constitué par l'ensemble des extrémités distales 5f de chaque fibre optique de transmission 4f. La figure 1B représente l'extrémité distale 5 du guide de lumière correspondant à configuration décrite sur la figure 1A.

Le guide de lumière est également apte à transmettre une lumière collectée par l'extrémité distale 5 jusqu'à l'extrémité proximale 3, à travers le tronçon central 4. Ainsi, l'extrémité distale 5 comporte un système optique distal 5a ayant une fonction de collection de rayonnements lumineux provenant du champ d'observation 10 situé en vis-à-vis de l'extrémité distale 5. Ainsi, le système optique distal 5a définit un angle solide d'observation Ω', dont l'intersection avec la surface de l'objet constitue le champ observé S_{Ω'}. La lumière collectée au niveau de l'extrémité distale 5, par le système optique distal 5a, peut être transférée par l'élément optique relais 4a jusqu'à un module de détection 3b, intégré à l'extrémité proximale 3. Le module de détection 3b a pour fonction la détection de la lumière collectée de l'extrémité distale 5 vers l'extrémité proximale 3.

Sous l'effet de l'illumination par le faisceau d'excitation 12, l'objet 10 émet une lumière d'émission 14 dans une bande spectrale d'émission λₑₘ. La lumière d'émission 14 peut être une partie du faisceau d'illumination 12 réfléchie ou rétrodiffusée par l'objet 10, la bande spectrale d'émission λₑₘ étant alors analogue à la bande spectrale d'excitationλₑₓ. Selon l'exemple représenté sur la figure 1A, la lumière d'émission 14 est une lumière de fluorescence, émise par l'objet, dans une longueur d'onde de fluorescence λ_{fluo} différente de la longueur d'onde d'excitation λₑₓ.

L'objet 10 comporte un ou plusieurs fluorophores endogènes ou exogènes. Dans le cas où les fluorophores sont endogènes, on parle d'autofluorescence. Les fluorophores exogènes sont préalablement injectés dans l'objet, de manière à se fixer spécifiquement sur des cibles, par exemple des cellules cancéreuses. Chaque fluorophore est apte à émettre un rayonnement de fluorescence 14, dans la bande spectrale de fluorescence λ_{fluo}, lorsqu'il est illuminé par un une lumière d'excitation 12, dans une bande spectrale d'excitation λₑₓ. Par exemple, lorsque le fluorophore utilisé est le vert d'indocyanine, ou ICG (acronyme anglais signifiant IndoCyanine Green), la bande spectrale d'excitation peut être comprise entre 750 nm et 800 nm, la bande spectrale de fluorescence étant comprise entre 820 nm et 870 nm.

Ainsi, sous l'effet du faisceau d'excitation transmis par l'extrémité distale 5 du guide 2, l'objet peut émettre une lumière de fluorescence 14. Dans l'exemple représenté, l'objet 10 comporte une zone fluorescente 13, apte à émettre une telle lumière de fluorescence 14. Une partie de cette dernière est collectée par le système optique distal 5a, puis transférée, à travers le tronçon relais 4, à un module de détection 3b, inclus dans l'extrémité proximale 3.

Le module de détection 3b comporte un séparateur spectral 6, apte à diriger un faisceau lumineux incident dans une direction donnée en fonction de sa bande spectrale. Le séparateur spectral 6 dirige la lumière d'émission 14 vers un capteur d'image d'émission 16. Le capteur d'image d'émission est apte à former une image *Iₑₘ* de la lumière d'émission 14 produite par l'objet 10 sous l'effet de l'illumination par le faisceau d'excitation 12 émanant de l'extrémité distale 5. Ce capteur d'image 16 sera détaillé par la suite.

Un processeur 50 est apte à traiter les images d'émission *Iₑₘ* formées par le capteur d'image 16. Il s'agit par exemple d'un microprocesseur intégré dans un ordinateur. En particulier, le processeur est un microprocesseur relié à une mémoire programmable dans laquelle est stockée une séquence d'instructions pour effectuer les opérations de traitement d'images. Ces opérations sont par exemple l'élimination de certains bruits de fond, la colorisation ou la superposition avec d'autres images, comme décrit ultérieurement.

Un écran 55, relié au processeur, permet la visualisation des images collectées par le capteur d'image 16 et traitées par le microprocesseur 50.

Selon une variante, représentée sur figure 1C, le tronçon central 4 ne comporte pas de fibre optique de transmission 4f, le faisceau d'excitation étant transmis par l'élément optique de relais 4a entre l'extrémité proximale 3 et l'extrémité proximale 5. Selon une telle variante, le système optique distal 5a assure la fonction d'élément projecteur du faisceau d'excitation sur l'objet 10, en définissant un angle solide de projection Ω. On comprend que selon cette variante, l'angle solide de projection Ω est égal à l'angle solide d'observation Ω', les champs éclairé et observé étant confondus.

### Mesure de distance

Le dispositif comporte également un capteur de distance 20. Ce dernier repose sur le principe bien connu de la télémétrie optique par temps de vol, qui consiste à évaluer la durée entre l'émission d'une impulsion lumineuse et la détection de cette impulsion. Cette durée est représentative de la distance parcourue par la lumière formant l'impulsion entre son émission et sa détection.

Le capteur de distance 20 comprend une source de lumière 21, dite source de lumière de télémétrie, et apte à émettre un faisceau lumineux de télémétrie 22 dans une bande spectrale de télémétrie λ_{d}. La source de lumière 21 est de préférence impulsionnelle. Elle émet des impulsions lumineuses, ou impulsions de télémétrie, à une fréquence impulsionnelle pouvant être comprise entre 1 Hz et 100 Hz voire 1 kHz. Dans cet exemple, la source de lumière 21 est une diode laser impulsionnelle émettant à une longueur d'onde λ_{d} = 950 nm. Elle est commandée par un processeur de télémétrie 51. La bande spectrale de télémétrie λ_{d} peut se situer en dessous de 400 nm ou en dessus de 900 nm, de façon à se situer ni dans le spectre visible, ni dans une bande spectrale d'émission (et notamment une bande spectrale de fluorescence). Ainsi, la lumière de télémétrie n'est pas détectée par les capteurs d'image visible ou d'émission.

Le faisceau lumineux de télémétrie 22 traverse un répartiteur de lumière 28, apte à diriger une première partie du faisceau de télémétrie vers le guide de lumière 2, à travers une fibre optique de télémétrie 21f, ou une pluralité de fibres optiques de télémétrie 21f. Chaque fibre de télémétrie est reliée au guide de lumière 2 par le coupleur optique 15, duquel débouche la fibre optique de couplage 15f. Cette fibre optique permet la transmission du faisceau d'excitation 12 et du faisceau de télémétrie 22 jusqu'à la fenêtre d'entrée 3a du guide de lumière. Le coupleur optique 15 permet de guider les faisceaux d'excitation et de télémétrie dans la même fibre optique ou dans un même faisceau de fibres optiques, jusqu'au guide de lumière 2.

Une deuxième partie du faisceau de télémétrie 22 est dirigée vers un photodétecteur de déclenchement 23. De préférence, la deuxième partie du faisceau de télémétrie est minoritaire par rapport à la première partie, et ne représente pas plus de 20% ou de 10% du faisceau émis par la source de télémétrie 21.

Le photodétecteur de déclenchement 23 est par exemple une photodiode, par exemple une photodiode à avalanche dont la bande passante est adaptée à la bande spectrale de télémétrie λ_{d}.

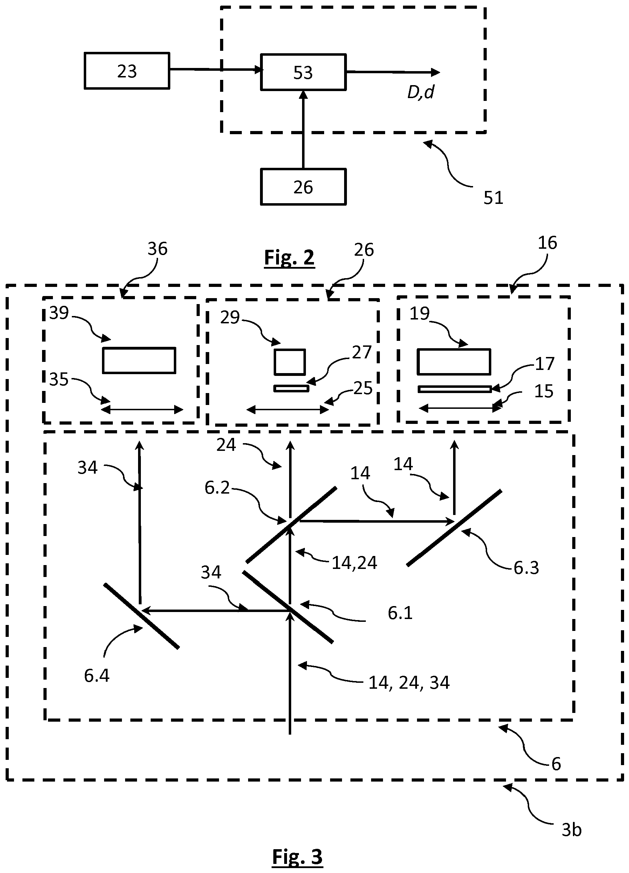

Comme représenté sur la figure 2, la détection d'une impulsion par le photodétecteur de déclenchement 23 enclenche, dans le processeur de télémétrie 51, l'activation de l'incrémentation d'un compteur de télémétrie 53 à une fréquence d'horloge, cette dernière s'élevant par exemple à 20 MHz. Ce compteur de télémétrie est inclus dans le processeur de télémétrie.

A l'instar du faisceau d'excitation 12, le faisceau de télémétrie 22 est dirigé vers l'objet 10 dans le guide de lumière 2 à travers la fenêtre d'entrée 3a. Il atteint alors l'extrémité distale 5 en étant transmis par les fibres de transmission 4f, à partir de l'extrémité desquelles il est émis en direction de l'objet 10 selon un cône d'émission identique, ou sensiblement similaire, au cône d'émission du faisceau d'excitation 12, d'angle solide de projection Ω.

Selon la variante représentée sur la figure 1C, le faisceau de télémétrie 22 est transmis jusqu'à l'extrémité distale 5 par le relais optique 4a et, de même que le faisceau d'excitation 12, est projeté sur l'objet par le système optique distal 5a.

Du fait de la différence de longueur d'onde du faisceau d'excitation 12 et le faisceau de télémétrie 22, des différences peuvent apparaître entre le cône d'émission du faisceau d'excitation et le cône d'émission du faisceau de télémétrie. Cependant, ces différences peuvent être négligées et on peut considérer que ces deux cônes d'émission ne forment qu'un seul cône définissant un même angle solide de projection Ω. C'est d'ailleurs un avantage remarquable de l'invention.

Selon une variante, la fibre optique d'excitation 11f et la fibre optique de télémétrie 21f s'étendent jusqu'à la fenêtre optique 3a. Cependant, il est préférable de disposer un coupleur optique 15 entre, d'une part, la source de lumière de télémétrie 12 et la source de lumière d'excitation 11 et, d'autre part, le guide de lumière 2. Le coupleur optique a pour fonction le couplage de la fibre optique d'excitation et de la fibre optique de télémétrie avec une fibre optique de couplage, cette dernière étant apte à transmettre les deux faisceaux jusqu'au guide de lumière. Ainsi, l'éclairement de l'objet, par l'un ou l'autre de ces faisceaux, est similaire, aussi bien au niveau de l'étendue que de la distribution spatiale de l'éclairement sur l'objet.

Une partie du faisceau de télémétrie 22 atteint l'objet 10 et est réfléchie par ce dernier, formant ainsi une lumière de télémétrie réfléchie 24. Une partie de cette lumière réfléchie 24 est collectée par le système optique distal 5a, puis est renvoyée par le relais optique 4a jusqu'au module de détection 3b, au niveau de l'extrémité proximale 3. Dans le module de détection 3b, le séparateur spectral 6 dirige la lumière de télémétrie réfléchie 24 vers un capteur de lumière de télémétrie 26. Ce capteur de lumière peut être par exemple une photodiode, et en particulier une photodiode à avalanche, de façon analogue au photodétecteur de déclenchement 23 précédemment décrit.

L'émission du faisceau lumineux de télémétrie 22 étant impulsionnelle, il en est de même pour la détection de la lumière de télémétrie réfléchie 24 par l'objet. Lorsqu'une impulsion est détectée par le capteur de lumière de télémétrie 26, l'incrémentation du compteur de télémétrie 53 est arrêtée. La valeur du compteur, c'est-à-dire un nombre d'incréments entre son activation, déclenchée par le photodétecteur de déclenchement 23, et son arrêt, déclenché par le capteur de lumière de télémétrie 26, permet de mesurer une distance D parcourue par l'impulsion de télémétrie 22, entre la source de lumière de télémétrie 21 et le photodétecteur de télémétrie 26 comme représenté sur la figure 2. Cette mesure peut être réalisée, par le processeur de télémétrie 51, en mettant en oeuvre un dispositif de type TDC, acronyme de Time to Digital Converter (convertisseur durée - valeur numérique). Ce processeur peut notamment être implanté dans un microcontrôleur de télémétrie.

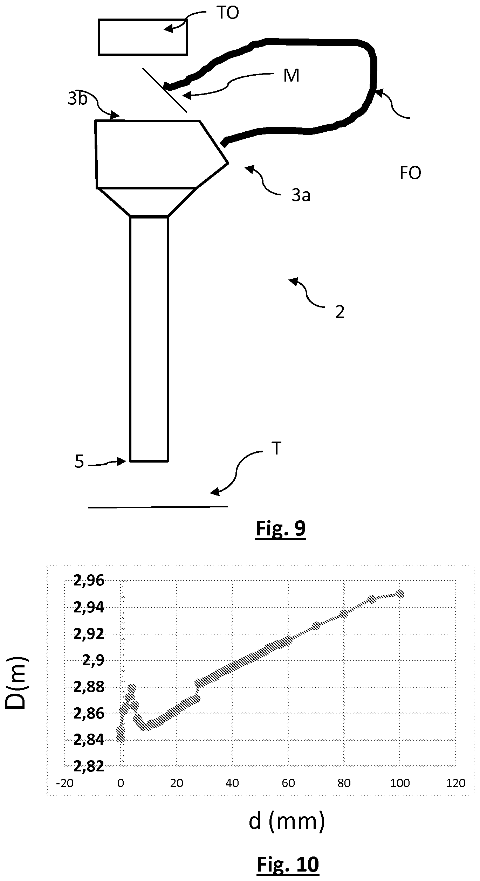

L'estimation de la distance d entre l'extrémité distale 5 du guide, et plus précisément de l'élément projecteur 5f, et l'objet 10 est réalisée par le processeur de télémétrie 51, en fonction des dimensions du guide de lumière, et en particulier les distances parcourues par la lumière de télémétrie respectivement entre la source de télémétrie 21 et l'extrémité distale 5 ainsi qu'entre l'extrémité distale 5 et le capteur de télémétrie 26. Cette détermination peut être réalisée sur la base d'un étalonnage, permettant d'estimer cette distance d en fonction de la mesure réalisée par le capteur de distance 20. Un exemple d'étalonnage est présenté sur la figure 10.

Il est également possible de mesurer une distance δ parcourue par le faisceau d'excitation 22 entre la source d'excitation 21 et l'objet 10.

Sur la base de l'estimation de la distance d entre l'élément projecteur 5f et l'objet 10, le processeur de télémétrie 51 adresse un signal de commande à un modulateur 18 pour moduler l'intensité du faisceau d'excitation émis par la source d'excitation 11, de façon à ce que la puissance délivrée par le faisceau d'excitation 12 sur l'objet soit inférieure à une puissance maximale autorisée Pmax, cela afin d'éviter tout risque de lésion de l'objet par le faisceau d'excitation, notamment lorsque l'objet est un tissu corporel. Par exemple, à λₑₓ = 750 nm, la puissance surfacique maximale s'élève à 0.25 W/cm². Cet ajustement de l'intensité est basé sur la distance d la plus proche, dans le champ d'observation, entre l'objet et l'extrémité distale 5 du guide d'excitation. A chaque distance d correspond une puissance surfacique maximale admissible Pmax_{d}, cette puissance maximale pouvant être déterminée expérimentalement, puis tabulée dans une mémoire.

L'ajustement de l'intensité du faisceau peut être réalisé en modulant un signal de commande de la source de lumière d'excitation. Il peut également être réalisé en disposant des atténuateurs, ou densités optiques, sur le parcours du faisceau d'excitation.

On remarque la similarité des trajets optiques du faisceau de télémétrie 22 et du faisceau d'excitation 12 dans le guide de lumière 2. Ces deux faisceaux sont projetés par l'élément projecteur 5f (ou, le cas échéant 5a) sur l'objet selon le même angle solide de projection Ω. De ce fait, et c'est un élément important de l'invention, la surface de l'objet éclairée par le faisceau d'excitation 12 correspond à la surface éclairée par le faisceau de télémétrie 22.

Aussi, la distance d mesurée dépend du point M de l'objet 10 le plus proche de l'extrémité distale 5. Cette propriété est particulièrement utile lorsque l'objet 10 n'est pas plan, et est susceptible de comporter, dans le même champ observé, des zones proches et des zones éloignées de l'extrémité distale 5. Parce que le faisceau de télémétrie 22 est réparti selon le même angle solide Ω que le faisceau d'excitation, la distance mesurée est la distance la plus proche, dans le champ observé, entre l'objet et l'extrémité distale 5 (ou entre l'objet et la source d'excitation 11). L'ajustement de la puissance du faisceau d'excitation est donc réalisé en se basant sur le point du champ observé et qui, recevant le faisceau d'excitation, est le plus proche de l'extrémité distale 5.

Cet aspect est illustré sur la figure 1D, représentant un objet dont la surface n'est pas plane. Cette surface est éclairée par les faisceaux d'excitation 12 et de télémétrie 22, par l'intermédiaire de l'élément projecteur 5f, sous le même angle solide de projection Ω. La lumière d'émission 14 (dans ce cas, une lumière de fluorescence) et la lumière de télémétrie 24 réfléchie par l'objet sont collectées, par le système optique distal 5a selon un même angle solide d'observation Ω'. Le point M correspond au point de la surface de l'objet le plus proche de l'extrémité distale 5. Suite à l'éclairement de la surface de l'objet par le faisceau de télémétrie 22, le point M est le premier point de la surface illuminé. De ce fait, le premier rayon de télémétrie réfléchi par l'objet est le rayon 24_{M}. Ce dernier est collecté par le système optique distal 5a, puis renvoyé vers le module de détection 3b, pour être detecté par le photodétecteur de télémétrie 26. Le compteur de télémétrie 53 est alors arrêté. La distance D mesurée, représentative du trajet optique du faisceau de télémétrie entre la source de télémétrie 21 et le photodétecteur de télémétrie 26, dépend donc du point de la surface de l'objet le plus proche de l'élément projecteur situé à l'extrémité distale 5 du guide de lumière 2, en l'occurrence le point M. Il en est de même pour la distance d déterminée, à partir de D, entre l'extrémité distale 5 et l'objet 10.

Quel que soit le mode de réalisation, l'angle solide de projection Ω est de préférence inférieur ou égal à l'angle solide d'observation Ω'. Avantageusement, l'angle solide de projection Ω est confondu avec l'angle solide d'observation Ω', de telle sorte que le champ éclairé S_{Ω} corresponde au champ observé S_{Ω'}. En effet, la mesure de distance est effectuée sur la surface de l'objet située dans la zone de recouvrement du champ éclairé S_{Ω} et du champ observé S_{Ω'}. L'aire du champ éclairé dépend de l'application visée. En endoscopie, elle est supérieure à 5 mm², voire à 1 cm², tandis qu'en imagerie de fluorescence peropératoire, elle est supérieure à 5 cm², voire à 10 cm².

Dans l'exemple représenté, le déclenchement d'une impulsion par la source de lumière de télémétrie 21 est commandé par le processeur de télémétrie 51.

Il est également possible, connaissant cette distance d, de moduler le faisceau d'excitation 12 de telle sorte que l'éclairage génère une puissance surfacique constante, dite puissance de consigne, au point de l'objet 10 le plus proche du guide de lumière, indépendamment de la position de l'extrémité distale 5 par rapport à l'objet 10. Pour cela, le modulateur 18 ajuste l'intensité du faisceau d'excitation par rapport à une distance de référence d_{ref} de telle sorte que quelle que soit la distance d entre l'extrémité distale et l'objet, la puissance surfacique P délivrée sur l'objet soit constante. Par exemple, si P_{ref} désigne la puissance de consigne, l'intensité du faisceau est modulée, connaissant la distance d, par une fonction de modulation f_{d}, dépendant de la distance, telle que f_{d}(P) = P_{ref}.

### Image visible

Le dispositif peut également comporter une source de lumière visible 31, apte à diriger une lumière visible 32, dans une bande spectrale visible λᵥᵢₛ, vers l'objet 10, à travers une fibre optique visible 31f et le guide de lumière 2.

La source de lumière visible 31 peut notamment être une source de lumière blanche, continue ou impulsionnelle. Dans cet exemple, la source de lumière visible est une diode électroluminescente blanche.

Une partie de la lumière visible est réfléchie par l'objet et est collectée par le système optique distal 5a pour être renvoyée vers l'extrémité proximale 3 du guide de lumière 2, et plus précisément vers le module de détection 3b. Le séparateur spectral 6 dirige la lumière visible réfléchie 34 vers un capteur d'image visible 36, ce dernier formant une image visible Iᵥᵢₛ.

Le processeur 50 est apte à traiter les images visibles *Iᵥᵢₛ* formées par le capteur d'image visible 36, en appliquant des opérations de traitement d'images. Une opération de traitement est par exemple la superposition avec une image d'émission *Iₑₘ* issue du capteur d'image d'émission 16, l'image d'émission étant préalablement colorisée. L'écran 55 permet la visualisation de l'image visible et/ou de l'image obtenue suite à la superposition.

Précisons que le déclenchement de la source de lumière de télémétrie 21 peut être réalisé de façon synchrone avec la source de lumière visible 31, ou de façon asynchrone. Lorsque la bande spectrale de télémétrie λ_{d} comporte des longueurs d'ondes de la bande spectrale visible λᵥᵢₛ, la source de lumière de télémétrie 21 est déclenchée de façon synchrone avec les instants auxquels la source de lumière visible 31 est éteinte.

### Module de détection

La figure 3 décrit un exemple de module de détection 3b, faisant partie de l'extrémité proximale 3 du guide de lumière 2.

Le séparateur spectral 6, précédemment évoqué, est destiné à renvoyer différents faisceaux lumineux vers un détecteur spécifique en fonction de leur longueur d'onde, en mettant en oeuvre des lames semi-réfléchissantes, ou lames dichroïques, transmettant la lumière dans une bande spectrale donnée et réfléchissant la lumière dans une autre bande spectrale.

Une première lame semi-réfléchissante 6.1 transmet la lumière dans la bande spectrale d'émission *λₑₘ* ainsi que dans la bande spectrale de télémétrie *λ_{d}*. Cette première lame réfléchit la lumière 34 dans la bande spectrale visible λᵥᵢₛ vers un miroir 6.4, ce dernier réfléchissant la lumière visible vers le capteur d'image visible 36. Ce capteur d'image comprend un photodétecteur matriciel 39, couplé à un système optique de focalisation 35.

Une deuxième lame semi-réfléchissante 6.2 transmet la lumière dans la bande spectrale de télémétrie λ_{d} vers un capteur de lumière de télémétrie 26 et réfléchit la lumière dans la bande spectrale d'émission *λₑₘ* vers un miroir 6.3, ce dernier réfléchissant la lumière d'émission 14 vers un capteur d'image d'émission 16. Ce capteur d'image d'émission 16 comprend un photodétecteur matriciel 19, couplé à un système optique de focalisation 15.

Lorsque la lumière d'émission est une lumière de fluorescence, la bande spectrale d'émission est une bande spectrale de fluorescence *λ_{fluo,}* le capteur d'image d'émission 16 étant un capteur d'image de fluorescence. Dans ce cas, de préférence, le capteur d'image de fluorescence 16 comporte un filtre de fluorescence 17, dont la bande passante est définie en fonction de la bande spectrale de fluorescence λ_{fluo}. La fonction de ce filtre de fluorescence est d'éviter la détection, par le capteur d'image de fluorescence, de rayonnements optiques non représentatifs de la fluorescence.

Les photodétecteurs matriciels visible 39 et d'émission 19 sont des capteurs de type CCD (acronyme anglais signifiant Charge Coupled Device - Dispositif à Transfert de Charge) de type CMOS (acronyme anglais signifiant Complementary Metal-Oxyde Semiconductor), voire un bolomètre, en particulier dans le cas d'une fluorescence dans une bande spectrale dans l'infrarouge.

Le capteur de lumière de télémétrie 26 comporte un photodétecteur de télémétrie 29 apte à la détection d'un rayonnement lumineux dans la bande spectrale de télémétrie λ_{d}. Il peut également comprendre un filtre de télémétrie 27 dont la bande passante est définie en fonction de la bande spectrale de télémétrie λ_{d}. La fonction de ce filtre de télémétrie est d'éviter la détection, de rayonnements optiques non représentatifs de la lumière de télémétrie 24 réfléchie par l'objet. Il peut également comprendre une optique de focalisation 25, notamment lorsque le photodétecteur de télémétrie 29 est un photodétecteur matriciel, ce cas de figure étant détaillé par la suite.

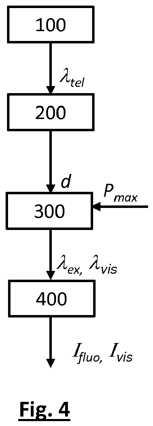

La figure 4 résume les principales étapes du procédé mis en oeuvre dans cet exemple :
Etape 100 : émission d'un faisceau de télémétrie 22, sous la forme d'une impulsion, par la source de lumière de télémétrie 21. Cette émission entraîne la détection de cette impulsion par le photodétecteur de déclenchement 23 et l'activation du compteur de télémétrie 53.
Etape 200 : détection d'une lumière de télémétrie 24 réfléchie par l'objet 10, par le capteur de télémétrie 26, ce qui entraîne l'arrêt du compteur de télémétrie 53 et la détermination de la distance D parcourue par la lumière de télémétrie entre la source de télémétrie 21 et le capteur de télémétrie 26.
Etape 300 : En fonction de cette distance D, détermination de la distance d entre l'extrémité distale 5 du guide 2 et l'objet, en fonction de quoi on effectue une modulation du faisceau d'excitation 12 émis par la source d'excitation 11, compte tenu d'une puissance d'éclairement Pₘₐₓ admissible par l'objet ou d'une puissance de consigne P_{ref}.
Etape 400 : émission du faisceau d'excitation 12 par la source d'excitation 11, et détection d'une image d'émission Iₑₘ, par exemple une image de fluorescence I_{fluo}, par le capteur d'image d'émission 16.

Cette étape peut également comprendre l'émission d'une lumière visible 32 par la source de lumière visible 31, et la détection d'une image visible Iᵥᵢₛ par le capteur d'image visible 36. L'utilisation de fibres optiques de transmission 4f, dans le guide de lumière 2, pour diriger le faisceau d'excitation 12 et le faisceau de télémétrie 22 vers l'objet 10 est jugée préférable à une configuration selon laquelle ces faisceaux sont dirigés vers l'objet par le relais optique 4a. En effet, selon cette dernière configuration, illustrée sur la figure 1C, des réflexions parasites peuvent se produire dans le relais optique 4a, induisant une potentielle détection intempestive d'une lumière de télémétrie par du capteur de télémétrie. Une telle détection intempestive se traduit par une mesure de distance erronée.

D'une façon générale, il est préférable que les chemins optiques empruntés par le faisceau d'excitation 12 et le faisceau de télémétrie 22 soient différents de ceux empruntés par la lumière d'émission 14 et la lumière de télémétrie 24 réfléchie par l'objet.

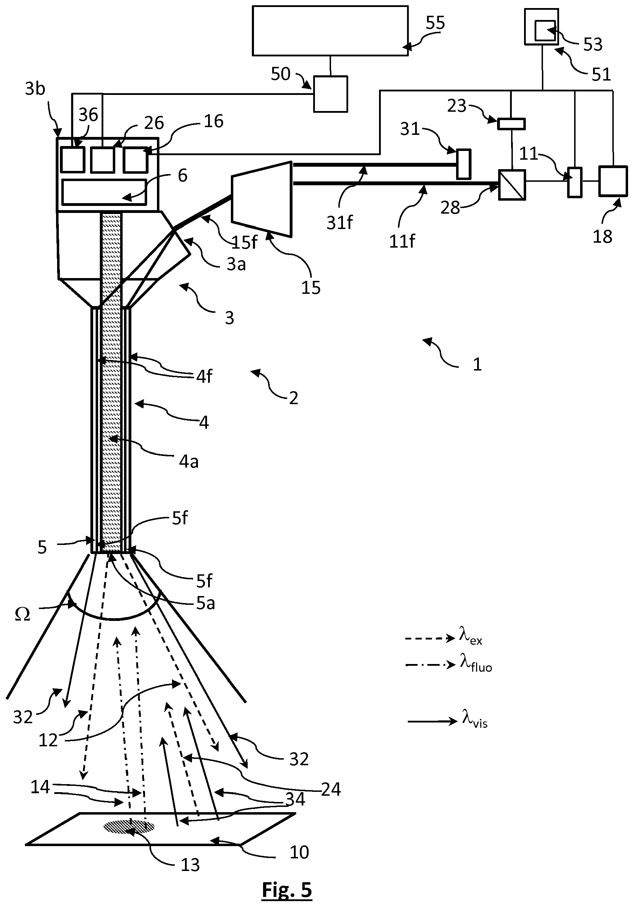

La figure 5 représente un autre mode de réalisation, selon lequel la source d'excitation 11 est impulsionnelle et constitue la source de lumière de télémétrie 21. Ainsi, la bande spectrale de télémétrie λ_{d} correspond à la bande spectrale d'excitation λₑₓ. Cela limite le nombre de sources de lumière mises en oeuvre dans le dispositif. Dans une telle configuration, la source de lumière d'excitation est de préférence impulsionnelle.

Une partie de ce faisceau est dirigée vers le photodétecteur de déclenchement 23 par le répartiteur 28, ce qui a pour effet l'activation du compteur de télémétrie 53. L'autre partie se propage vers l'objet 10 à travers le guide de lumière 2. L'objet réfléchit une partie du faisceau d'excitation et cette lumière d'excitation (ou de télémétrie) 24 réfléchie par l'objet est transmise jusqu'au capteur de télémétrie 26.

Certains éléments du capteur de distance 20 peuvent être intégrés dans le guide de lumière, par exemple au niveau du module de détection 3b de l'extrémité proximale 3.

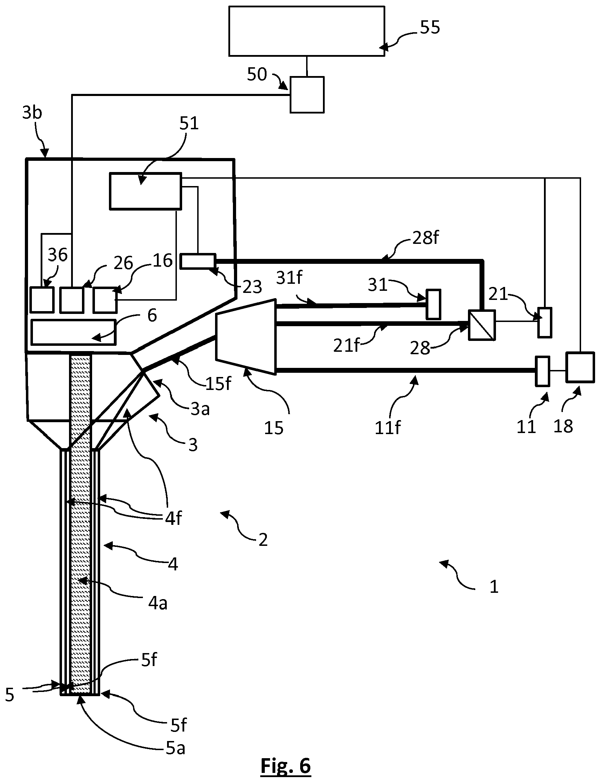

La figure 6 représente un exemple d'une telle intégration. Dans cette figure, le photodétecteur de déclenchement 23, est intégré dans le module de détection 3b, de même que le processeur de télémétrie 51. Cela suppose une liaison par une fibre optique 28f entre le répartiteur 28, et le photodétecteur de déclenchement 23. Ce mode de réalisation permet de réduire la longueur des connexions électriques entre les éléments clefs du capteur de distance 20, à savoir le photodétecteur de déclenchement 23, le capteur de télémétrie 26 et le processeur de télémétrie 51. Cela améliore la précision de la mesure.

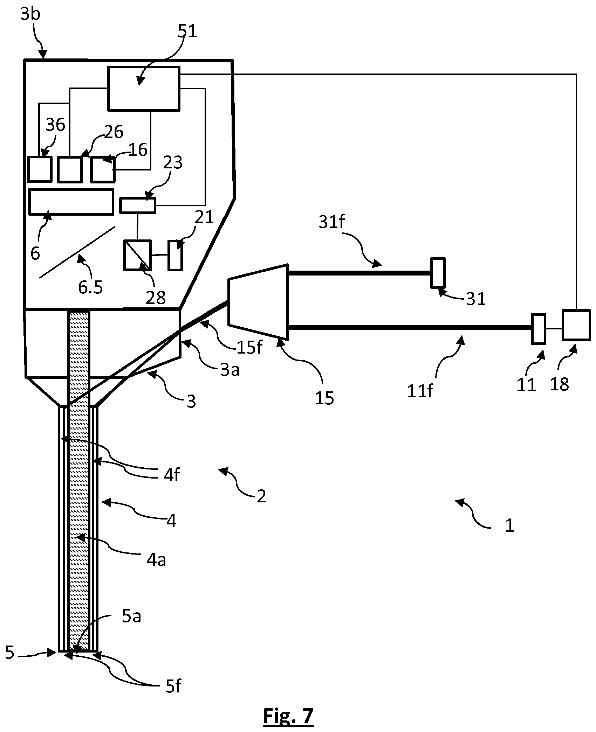

Selon une autre variante, représentée sur la figure 7, la source de lumière de télémétrie 21, le répartiteur 28, le photodétecteur de déclenchement 23 et le processeur sont intégrés dans le module de détection 3b, faisant partie de l'extrémité proximale 3 du guide de lumière 2. Un miroir semi-réfléchissant 6.5 permet de diriger le faisceau de lumière de télémétrie 22, produit par la source de lumière de télémétrie 21, vers l'extrémité distale 5 du guide de lumière, à travers l'élément optique de relais 4a. Ce miroir semi-réfléchissant permet également la transmission de la lumière d'émission 14, ou des lumières de télémétrie 24 ou visible 34 réfléchies par l'objet 10, se propageant depuis l'extrémité distale 5, vers le séparateur spectral 6. Cette variante permet une intégration complète du capteur de mesure de distance 20 dans l'extrémité proximale du guide de lumière 2. Cela permet d'obtenir un dispositif particulièrement compact.

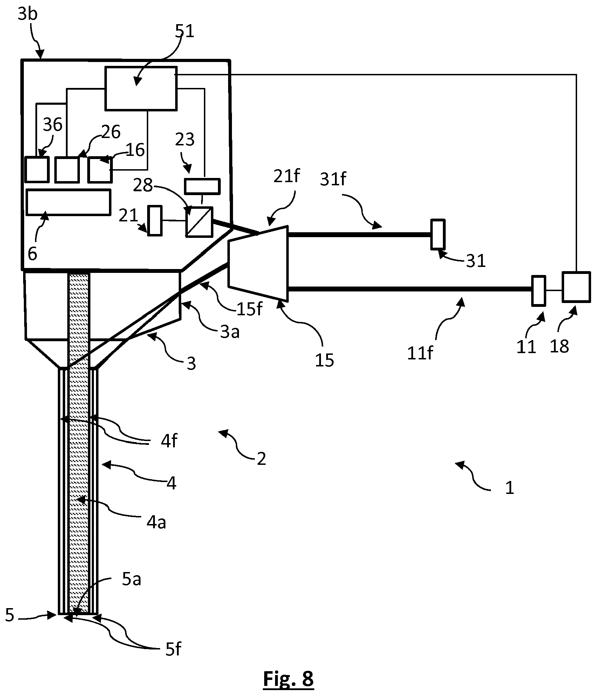

La variante représentée sur la figure 8 est similaire à celle représentée sur la figure 7, la transmission du faisceau de télémétrie 22 entre la source de lumière de télémétrie 21 et l'objet 10 étant réalisée par les fibres optiques d'excitation 21f, couplage 15f, et de transmission 4f.

Dans les configurations représentées sur la figure 7 ou la figure 8, la source d'excitation 11 peut également être logée dans le guide 2, notamment au niveau de son extrémité proximale 3.

Un essai expérimental a été réalisé, utilisant le dispositif représenté sur la figure 9. Un télémètre optique commercial TO est placé face à un miroir M, en face duquel est disposée une fibre optique FO, cette dernière étant raccordée à la fenêtre d'entrée 3a d'un laparoscope 2. Le télémètre émet un faisceau lumineux de télémétrie apte à être collecté par la fibre optique FO, et transmis par le laparoscope jusqu'à une cible T. Le télémètre optique TO comprend un photodétecteur, ce dernier étant disposé face au module de détection 3b du laparoscope. Le photodétecteur du télémètre détecte la lumière de télémétrie réfléchie par la cible T, transmise par le laparoscope. Dans cet exemple, le module de détection est constitué d'une simple fenêtre transparente. La longueur de la fibre optique FO s'élève à 2.5 m.

La figure 10 représente la distance D mesurée par le télémètre TO, correspondant au trajet optique du faisceau lumineux de télémétrie, en fonction de la distance d entre l'extrémité distale 5 du laparoscope 2 et la cible T. Cette figure montre que la mesure de la distance mesurée D permet une estimation précise de la distance d dès lors que l'extrémité distale 5 du laparoscope 2 est éloignée de plus de 15 mm de l'objet. Les fluctuations apparaissant lorsque d < 15 mm sont dues à un éclairement inhomogène de la cible lorsque cette dernière est placée trop proche de l'extrémité distale.

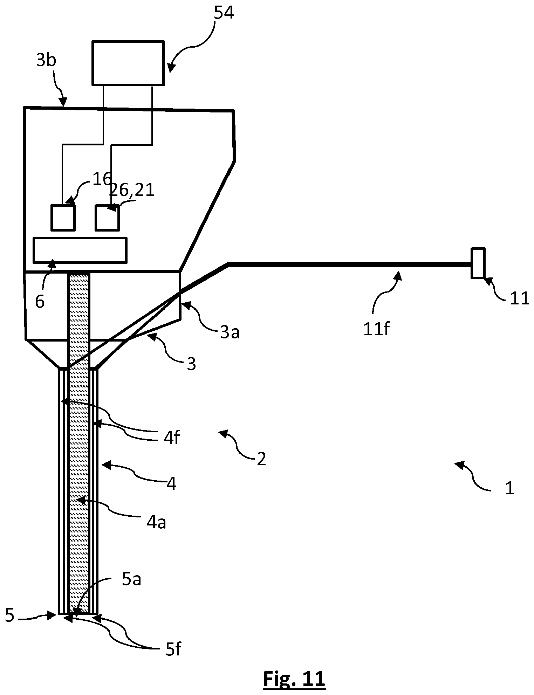

La figure 11 représente un autre mode de réalisation dans lequel la surface de l'objet est divisée en une pluralité de surfaces élémentaires. Selon ce mode de réalisation, on ne cherche pas à établir la distance la plus proche entre l'extrémité distale 5 du guide de lumière 2 et la surface de l'objet, comme dans les modes de réalisations précédemment décrits, mais à obtenir les distances entre l'extrémité distale 5 et chaque élément de surface.

Le capteur de télémétrie 26 comporte un photodétecteur 29 matriciel, dont chaque pixel est apte à établir une mesure de la distance le séparant de l'élément de surface dont il est optiquement couplé, c'est-à-dire de l'élément de surface conjugué de ce pixel. Un tel photodétecteur peut par exemple être une caméra 3D à temps de vol, par exemple le modèle SR4000 commercialisé par la société Mesa Imaging.

Une telle caméra comporte une source impulsionnelle de lumière de télémétrie 21, par exemple sous la forme d'une diode électroluminescente émettant dans une bande spectrale λ_{d} centrée sur la longueur d'onde 850 nm. Le faisceau de lumière de télémétrie est dirigé vers l'objet 10 à travers le relais optique 4a, puis le système optique distal 5a. La lumière de télémétrie réfléchie est collectée par le système optique distal 5a, puis renvoyée vers le photodétecteur 29 à travers le relais optique 4a.

Le photodétecteur est apte à déterminer la durée écoulée entre l'impulsion de lumière de télémétrie 22 par la source de lumière et la détection de cette impulsion sur chacun de ces pixels. Cela permet d'obtenir une mesure de la distance parcourue par la lumière de télémétrie entre l'objet et chaque pixel du capteur de lumière de télémétrie 26, de laquelle on peut estimer la distance entre l'extrémité distale 5 du guide 2 et chaque élément de surface.

Un tel dispositif permet alors d'obtenir une représentation tridimensionnelle de l'objet 10 situé face à l'extrémité distale 5.

Il peut permettre la préparation ou le suivi d'interventions délicates, requérant une information précise des dimensions de l'objet. Il peut par exemple s'agir d'interventions chirurgicales, dans lesquels certains organes sensibles ne doivent pas être touchés par des outils chirurgicaux. Les données tridimensionnelles relatives au tissu corporel examiné peuvent être transmises à une interface haptique, afin de fournir une assistance au geste chirurgical, qu'il soit robotisé ou manuel. Cette interface haptique peut notamment alerter le chirurgien lorsqu'un outil chirurgical se trouve à proximité d'un organe sensible. On comprend alors l'intérêt de disposer de données tridimensionnelles du champ observé en temps réel.

De préférence, le dispositif représenté sur la figure 11 comporte une source de lumière d'excitation, telle que précédemment décrite, apte à générer un faisceau d'excitation 12. Le faisceau d'excitation est guidé par le guide de lumière 2 jusqu'à l'extrémité distale 5. La lumière d'émission émise par l'objet suite à cette excitation est alors collectée par le système optique distal 5a, et renvoyée vers le capteur d'image d'émission 16. Un processeur 54 peut permettre le traitement ou l'affichage des données tridimensionnelles transmises par le capteur d'image de télémétrie 26 ainsi que de l'image générée par le capteur d'image d'émission 16.

La source de lumière d'excitation 11 peut être remplacée par une source de lumière visible 31. De même, le capteur d'image d'émission 16 peut être remplacé à un capteur d'image visible 36. Le dispositif peut notamment intégrer les caractéristiques des dispositifs présentés dans les modes de réalisation précédents dès lors qu'elles sont techniquement compatibles.

Au-delà des laparoscopes, l'invention s'applique à tout endoscope ou, de façon générale, à tout dispositif d'acquisition d'une image, notamment une image de fluorescence, en réponse à une excitation lumineuse.

## Revendications

1. Dispositif (1) d'observation d'un objet (10) comportant :
- une source de lumière d'excitation (11) apte à produire un faisceau d'excitation (12), dans une bande spectrale d'excitation (λₑₓ) se propageant vers un objet,
- un capteur d'image d'émission (16), apte à collecter une lumière d'émission (14) émise par l'objet (10), sous l'effet dudit faisceau d'excitation (12), dans une bande spectrale d'émission (λₑₘ, λ_{fluo}), le capteur d'image d'émission étant apte à acquérir une image d'émission (*Iₑₘ, I_{fluo}*) à partir de la lumière d'émission collectée,
- un capteur de distance (20) comportant une source de lumière de télémétrie (21), apte à émettre un faisceau de télémétrie (22), dans une bande spectrale de télémétrie (λ_{d}), se propageant vers l'objet (10),
- le capteur de distance comportant également un capteur de lumière de télémétrie (26), apte à détecter une lumière de télémétrie (24) réfléchie par l'objet (10), dans la bande spectrale de télémétrie (λ_{d}),
le dispositif étant **caractérisé en ce qu'**il comporte :
- un élément projecteur (5a, 5f), configuré pour projeter ledit faisceau d'excitation (12) et ledit faisceau de télémétrie (22), vers l'objet, selon un même angle solide de projection (Ω),
- le capteur de distance (20) étant apte à mesurer une distance (d) la plus courte, dans l'angle solide de projection, entre l'élément projecteur (5a, 5f) et l'objet, en fonction d'une durée entre l'émission du faisceau de télémétrie (22) par la source de lumière de télémétrie, et la détection, par le capteur de lumière de télémétrie, de la lumière de télémétrie (24) réfléchie par l'objet,
- un modulateur (18), configuré pour moduler une intensité du faisceau d'excitation (12), en fonction de ladite distance (d) mesurée par le capteur de distance (20).

2. Dispositif d'observation selon la revendication 1, comportant un guide de lumière (2), s'étendant entre une extrémité proximale (3) et une extrémité distale (5), le guide de lumière étant apte à transmettre :
- le faisceau d'excitation (12) et le faisceau de télémétrie (22) de l'extrémité proximale (3) vers l'extrémité distale (5),
- ladite lumière d'émission (14) et ladite lumière de télémétrie émises par l'objet (24) de l'extrémité distale (5) vers l'extrémité proximale (3),
l'extrémité distale (5) dudit guide de lumière (2) comportant ledit élément projecteur (5a, 5f).

3. Dispositif d'observation selon la revendication 2, dans lequel le guide de lumière comporte, au niveau de son extrémité distale (5), un système optique (5a) apte à collecter la lumière d'émission (14) et la lumière de télémétrie (24) provenant de l'objet (10), de façon à les diriger vers l'extrémité proximale (3) du guide de lumière.

4. Dispositif selon la revendication 3, dans lequel ledit système optique (5a) est apte à diriger le faisceau d'excitation (12) et le faisceau de télémétrie (22) vers l'objet (10), le système optique (5a) formant alors l'élément projecteur.

5. Dispositif selon la revendication 2 ou la revendication 3, dans lequel :
- le guide de lumière (2) comporte au moins une fibre optique de transmission (4f), s'étendant entre l'extrémité proximale (3) et l'extrémité distale du guide de lumière (2), de façon à guider le faisceau d'excitation et le faisceau de télémétrie entre l'extrémité proximale (3) et l'extrémité distale (5),
- l'extrémité de chaque fibre optique de transmission (4f) au niveau de l'extrémité distale (5) formant l'élément projecteur (5f).

6. Dispositif selon l'une quelconque des revendications 2 à 5, dans lequel le guide de lumière comporte un séparateur spectral (6), apte à diriger :
- la lumière d'émission (14) vers le capteur d'image d'émission (16),
- la lumière de télémétrie (24) réfléchie par l'objet (10) vers le capteur de lumière de télémétrie (26).

7. Dispositif selon l'une quelconque des revendications 2 à 6 comportant :
- une source de lumière visible (31), apte à émettre un faisceau lumineux visible (32) vers l'objet (10) dans une bande spectrale visible (λᵥᵢₛ),
- un capteur d'image visible (36), apte à collecter une lumière visible (34) réfléchie par l'objet sous l'effet d'une illumination par le faisceau lumineux visible (32), le capteur d'image visible étant apte à acquérir une image visible (*Iᵥᵢₛ*) à partir de la lumière visible collectée,
et dans lequel le guide de lumière (2) est apte à transmettre :
- le faisceau lumineux visible (32) de l'extrémité proximale (3) vers l'extrémité distale (5)
- et la lumière visible (34) réfléchie par l'objet (10) de l'extrémité distale (5) vers l'extrémité proximale (3).

8. Dispositif selon l'une quelconque des revendications précédentes, dans lequel la source de lumière de télémétrie (21) est confondue avec la source de lumière d'excitation (11).

9. Dispositif selon l'une quelconque des revendications précédentes, dans lequel le capteur de distance (20) comporte :
- un répartiteur (28), apte à renvoyer une partie du faisceau de télémétrie (22), émis par la source de télémétrie (22), vers un photodétecteur de déclenchement (23), ce dernier étant apte à détecter ladite partie du faisceau de télémétrie renvoyée par ledit répartiteur (28),
- un processeur de télémétrie (51), apte à déterminer une distance (D) de parcours du faisceau de télémétrie (22), entre la source de lumière de télémétrie (21) et le capteur de télémétrie (26), en fonction d'un instant de déclenchement, auquel le photodétecteur de déclenchement (23) détecte le faisceau de télémétrie (22) et d'un instant d'arrêt, auquel le capteur de lumière de télémétrie (26) détecte la lumière de télémétrie réfléchie par l'objet,
- le processeur de télémétrie (51) étant apte à déterminer la distance (d) entre l'élément projecteur (5a, 5f) et l'objet (10) en fonction de la distance (D) de parcours du faisceau de télémétrie.

10. Procédé d'observation d'un objet comportant les étapes suivantes :
- illumination de l'objet (10) par un faisceau lumineux de télémétrie (22), émis par une source de lumière de télémétrie (21), dans une bande spectrale de télémétrie (λ_{d}), le faisceau de télémétrie étant projeté sur l'objet par un élément projecteur (5a, 5f), selon un angle solide de projection (Ω),
- détection d'une lumière de télémétrie (24) réfléchie par l'objet, dans ladite bande spectrale de télémétrie (λ_{d}),
- en fonction d'une durée entre l'émission du faisceau de télémétrie (22) et la détection de la lumière de télémétrie (24) réfléchie par l'objet, mesure d'une distance (d) entre l'élément projecteur (5a, 5f) et l'objet (10),
- illumination dudit objet (10) à l'aide d'un faisceau d'excitation (12) émis par une source de lumière d'excitation (11), dans une bande spectrale d'excitation (λₑₓ), le faisceau d'excitation étant projeté sur l'objet par l'élément projecteur (5a, 5f), selon l'angle solide de projection (Ω),
- détection d'une lumière d'émission (14) par un capteur d'image d'émission (16), la lumière d'émission (14) étant émise par l'objet (10) sous l'effet de l'illumination par le faisceau d'excitation (12),
- acquisition d'une image d'émission (I*ₑₘ*, *I_{fluo}*) par ledit capteur d'image d'émission (16) à
partir de ladite lumière d'émission (14) détectée,
le procédé étant **caractérisé en ce qu'**il comporte également :
- la modulation d'une intensité du faisceau d'excitation (12), en fonction de la distance (d) mesurée, la distance mesurée correspondant à la distance la plus courte, dans l'angle solide de projection (Ω), entre l'élément projecteur (5a, 5f) et l'objet.

11. Procédé selon la revendication 10, dans lequel :
- le faisceau d'excitation (12) et le faisceau lumineux de télémétrie (22) sont transmis jusqu'à l'objet (10) par un guide de lumière (2), dont une extrémité distale (5), comprenant l'élément projecteur (5a, 5f), est placée face à l'objet (10),
- le guide de lumière assurant également une transmission de la lumière d'émission (14) émise par l'objet et de la lumière de télémétrie (24) réfléchie par l'objet respectivement vers le capteur d'image d'émission (16) et le capteur de lumière de télémétrie (26).

12. Procédé selon la revendication 11, dans lequel la lumière d'émission (14) émise par l'objet et la lumière de télémétrie (24) réfléchie par l'objet, sont collectées par un système optique (5a) situé à l'extrémité distale (5) du guide de lumière (2), avant d'être renvoyées vers le capteur d'image d'émission (16) et le capteur de lumière de télémétrie (26).

13. Procédé selon la revendication 12, dans lequel le faisceau d'excitation (12) et le faisceau de télémétrie (14) sont projetés sur l'objet (10) par le système optique (5a), ce dernier formant ledit élément projecteur (5a).

14. Procédé selon la revendication 11, dans lequel le faisceau d'excitation (12) et le faisceau de télémétrie (14) sont projetés sur l'objet (10) par au moins fibre optique (4f), s'étendant entre l'extrémité proximale (3) et l'extrémité distale (5) du guide de lumière (2), l'extrémité de chaque fibre optique au niveau de ladite extrémité distale (5) formant l'élément projecteur (5f).

15. Procédé selon l'une quelconque des revendications 10 à 14, dans lequel la lumière d'émission est une lumière de fluorescence, dans une bande spectrale de fluorescence (λ_{fluo}) différente de la bande spectrale d'excitation (λₑₓ).

16. Procédé selon l'une quelconque des revendications 10 à 15 comportant également :
- l'illumination de l'objet par un faisceau lumineux visible (32) produit par une source de lumière visible (31), dans une bande spectrale visible (λᵥᵢₛ), à travers le guide de lumière (2) ;
- l'acquisition d'une image visible (*Iᵥᵢₛ*) de l'objet (10) à l'aide d'un capteur d'image visible (36), ce dernier détectant une lumière visible réfléchie par l'objet (34) à travers le guide de lumière (2).

## Patentansprüche

1. Vorrichtung (1) zur Beobachtung eines Objekts (10), umfassend:
- eine Anregungslichtquelle (11) die geeignet ist, einen Anregungsstrahl (12) in einem Anregungsspektralband (λₑₓ) zu erzeugen, der sich zu einem Objekt hin ausbreitet,
- einen Emissionsbildsensor (16), der geeignet ist, ein vom Objekt (10) unter der Wirkung des Anregungsstrahls (12) emittiertes Emissionslicht (14) in einem Emissionsspektralband (λₑₘ, λ_{fluo}) zu sammeln, wobei der Emissionsbildsensor geeignet ist, ausgehend von dem gesammelten Emissionslicht ein Emissionsbild (I*ₑₘ*, I*_{fluo}*) zu erfassen,
- einen Abstandssensor (20), der eine Telemetrielichtquelle (21) umfasst, die geeignet ist, einen Telemetriestrahl (22) in einem Telemetriespektralband (λ_{d}) zu emittieren, der sich zum Objekt (10) hin ausbreitet,
- wobei der Abstandssensor ferner einen Telemetrielichtsensor (26) umfasst, der geeignet ist, ein vom Objekt (10) reflektiertes Telemetrielicht (24) im Telemetriespektralband (λ_{d}) zu detektieren,
wobei die Vorrichtung **dadurch gekennzeichnet ist, dass** sie umfasst:
- ein Projektorelement (5a, 5f), das dazu ausgestaltet ist, den Anregungsstrahl (12) und den Telemetriestrahl (22) gemäß demselben Projektionsraumwinkel (Ω) zum Objekt hin zu projizieren,
- wobei der Abstandssensor (20) geeignet ist, einen kürzesten Abstand (d), im Projektionsraumwinkel, zwischen dem Projektorelement (5a, 5f) und dem Objekt in Abhängigkeit von einer Dauer zwischen der Emission des Telemetriestrahls (22) durch die Telemetrielichtquelle und der Detektion des vom Objekt reflektierten Telemetrielichts (24) durch den Telemetrielichtsensor zu messen,
- einen Modulator (18), der dazu ausgestaltet ist, eine Intensität des Anregungsstrahls (12) in Abhängigkeit von dem vom Abstandssensor (20) gemessenen Abstand (d) zu modulieren.

2. Vorrichtung nach Anspruch 1, umfassend einen Lichtleiter (2), der sich zwischen einem proximalen Ende (3) und einem distalen Ende (5) erstreckt, wobei der Lichtleiter geeignet ist, Folgendes zu übertragen:
- den Anregungsstrahl (12) und den Telemetriestrahl (22) vom proximalen Ende (3) zum distalen Ende (5),
- das Emissionslicht (14) und das Telemetrielicht, die vom Objekt (24) emittiert werden, vom distalen Ende (5) zum proximalen Ende (3),
wobei das distale Ende (5) des Lichtleiters (2) das Projektorelement (5a, 5f) umfasst.

3. Vorrichtung nach Anspruch 2, bei welcher der Lichtleiter an seinem distalen Ende (5) ein optisches System (5a) umfasst, das geeignet ist, das Emissionslicht (14) und das Telemetrielicht (24), die vom Objekt (10) kommen, so zu sammeln, dass sie zum proximalen Ende (3) des Lichtleiters gelenkt werden.

4. Vorrichtung nach Anspruch 3, bei der das optische System (5a) geeignet ist, den Anregungsstrahl (12) und den Telemetriestrahl (22) zum Objekt (10) zu lenken, wobei das optische System (5a) dann das Projektorelement bildet.

5. Vorrichtung nach Anspruch 2 oder Anspruch 3, bei welcher:
- der Lichtleiter (2) mindestens eine optische Übertragungsfaser (4f) umfasst, die sich zwischen dem proximalen Ende (3) und dem distalen Ende des Lichtleiters (2) erstreckt, so dass der Anregungsstrahl und der Telemetriestrahl zwischen dem proximalen Ende (3) und dem distalen Ende (5) geleitet werden,
- das Ende jeder optischen Übertragungsfaser (4f) am distalen Ende (5) das Projektorelement (5f) bildet.

6. Vorrichtung nach einem der Ansprüche 2 bis 5, bei welcher der Lichtleiter einen Spektralteiler (6) umfasst, der geeignet ist, Folgendes zu lenken:
- das Emissionslicht (14) zum Emissionsbildsensor (16),
- das vom Objekt (10) reflektierte Telemetrielicht (24) zum Telemetrielichtsensor (26).

7. Vorrichtung nach einem der Ansprüche 2 bis 6, umfassend:
- eine Quelle sichtbaren Lichts (31), die geeignet ist, einen Strahl sichtbaren Lichts (32) in einem sichtbaren Spektralband (λᵥᵢₛ) zum Objekt (10) hin zu emittieren,
- einen Sensor für sichtbare Bilder (36), der geeignet ist, ein sichtbares Licht (34), das vom Objekt unter der Wirkung einer Beleuchtung durch den Strahl sichtbaren Lichts (32) reflektiert wird, zu sammeln, wobei der Sensor für sichtbare Bilder geeignet ist, ausgehend von dem gesammelten sichtbaren Licht ein sichtbares Bild (Iᵥᵢₛ) zu erfassen,
und bei welcher der Lichtleiter (2) geeignet ist, Folgendes zu übertragen:
- den Strahl sichtbaren Lichts (32) vom proximalen Ende (3) zum distalen Ende (5),
- und das vom Objekt (10) reflektierte sichtbare Licht (34) vom distalen Ende (5) zum proximalen Ende (3).

8. Vorrichtung nach einem der vorhergehenden Ansprüche, bei welcher die Telemetrielichtquelle (21) mit der Anregungslichtquelle (11) zusammenfällt.

9. Vorrichtung nach einem der vorhergehenden Ansprüche, bei welcher der Abstandssensor (20) Folgendes umfasst:
- einen Verteiler (28), der geeignet ist, einen Teil des Telemetriestrahls (22), der von der Telemetriequelle (22) emittiert wird, zu einem Auslöse-Fotodetektor (23) weiterzuleiten, wobei Letzterer geeignet ist, den vom Verteiler (28) weitergeleiteten Teil des Telemetriestrahls zu detektieren,
- einen Telemetrieprozessor (51), der geeignet ist, eine zurückgelegte Strecke (D) des Telemetriestrahls (22) zwischen der Telemetrielichtquelle (21) und dem Telemetriesensor (26) in Abhängigkeit von einem Auslösezeitpunkt, zu dem der Auslöse-Fotodetektor (23) den Telemetriestrahl (22) detektiert, und einem Ausschaltezeitpunkt, zu dem der Telemetrielichtsensor (26) das vom Objekt reflektierte Telemetrielicht detektiert, zu bestimmen,
- wobei der Telemetrieprozessor (51) geeignet ist, den Abstand (d) zwischen dem Projektorelement (5a, 5f) und dem Objekt (10) in Abhängigkeit von der zurückgelegten Strecke (D) des Telemetriestrahls zu bestimmen.

10. Verfahren zum Beobachten eines Objekts, umfassend die folgenden Schritte:
- Beleuchten des Objekts (10) durch einen Telemetrielichtstrahl (22), der von einer Telemetrielichtquelle (21) emittiert wird, in einem Telemetriespektralband (λ_{d}), wobei der Telemetriestrahl von einem Projektorelement (5a, 5f) gemäß einem Projektionsraumwinkel (Ω) auf das Objekt projiziert wird,
- Detektieren eines Telemetrielichts (24), das vom Objekt reflektiert wird, in dem Telemetriespektralband (λ_{d}),
- Messen eines Abstands (d) zwischen dem Projektorelement (5a, 5f) und dem Objekt (10) in Abhängigkeit von einer Dauer zwischen der Emission des Telemetriestrahls (22) und der Detektion des vom Objekt reflektierten Telemetrielichts (24),
- Beleuchten des Objekts (10) mithilfe eines Anregungsstrahls (12), der von einer Anregungslichtquelle (11) emittiert wird, in einem Anregungsspektralband (λₑₓ), wobei der Anregungsstrahl vom Projektorelement (5a, 5f) gemäß einem Projektionsraumwinkel (Ω) auf das Objekt projiziert wird,
- Detektieren eines Emissionslichts (14) durch einen Emissionsbildsensor (16), wobei das Emissionslicht (14) vom Objekt (10) unter der Wirkung der Beleuchtung durch den Anregungsstrahl (12) emittiert wird,
- Erfassen eines Emissionsbildes (I*ₑₘ*, I*_{fluo}*) durch den Emissionsbildsensor (16) ausgehend von dem detektierten Emissionslicht (14),
wobei das Verfahren **dadurch gekennzeichnet ist, dass** es ferner umfasst:
- das Modulieren einer Intensität des Anregungsstrahls (12) in Abhängigkeit von dem gemessenen Abstand (d), wobei der gemessene Abstand dem kürzesten Abstand, im Projektionsraumwinkel (Ω), zwischen dem Projektorelement (5a, 5f) und dem Objekt entspricht.

11. Verfahren nach Anspruch 10, bei dem:
- der Anregungsstrahl (12) und der Telemetrielichtstrahl (22) bis zum Objekt (10) von einem Lichtleiter (2) übertragen werden, von dem ein distales Ende (5), welches das Projektorelement (5a, 5f) umfasst, gegenüber dem Objekt (10) angeordnet ist,
- wobei der Lichtleiter ferner eine Übertragung des vom Objekt emittierten Emissionslichts (14) und des vom Objekt reflektierten Telemetrielichts (24) zum Emissionsbildsensor (16) beziehungsweise zum Telemetrielichtsensor (26) gewährleistet.

12. Verfahren nach Anspruch 11, bei dem das vom Objekt emittierte Emissionslicht (14) und das vom Objekt reflektierte Telemetrielicht (24) von einem optischen System (5a) gesammelt werden, das am distalen Ende (5) des Lichtleiters (2) gelegen ist, bevor sie zum Emissionsbildsensor (16) und zum Telemetrielichtsensor (26) weitergeleitet werden.

13. Verfahren nach Anspruch 12, bei dem der Anregungsstrahl (12) und der Telemetriestrahl (14) auf das Objekt (10) durch das optische System (5a) projiziert werden, wobei Letzteres das Projektorelement (5a) bildet.

14. Verfahren nach Anspruch 11, bei dem der Anregungsstrahl (12) und der Telemetriestrahl (14) auf das Objekt (10) durch mindestens eine optische Faser (4f) projiziert werden, die sich zwischen dem proximalen Ende (3) und dem distalen Ende (5) des Lichtleiters (2) erstreckt, wobei das Ende jeder optischen Faser am distalen Ende (5) das Projektorelement (5f) bildet.

15. Verfahren nach einem der Ansprüche 10 bis 14, bei dem das Emissionslicht ein Fluoreszenzlicht in einem Fluoreszenzspektralband (λ_{fluo}) ist, das verschieden vom Anregungsspektralband (λₑₓ) ist.

16. Verfahren nach einem der Ansprüche 10 bis 15, umfassend ferner:
- das Beleuchten des Objekts durch einen Strahl sichtbaren Lichts (32), der von einer Quelle sichtbaren Lichts erzeugt wird (31), in einem sichtbaren Spektralband (λᵥᵢₛ), durch den Lichtleiter (2) hindurch;
- das Erfassen eines sichtbaren Bildes (*Iᵥᵢₛ*) des Objekts (10) mithilfe eines Sensors für sichtbare Bilder (36), wobei Letzterer ein sichtbares Licht detektiert, das vom Objekt (34) durch den Lichtleiter (2) hindurch reflektiert wird.

## Claims

1. Device (1) for observing an object (10), including:
- an excitation-light source (11) able to produce, in an excitation spectral band (λₑₓ), an excitation beam (12) that propagates toward an object;
- an emission-image sensor (16) that is able to collect emission light (14) emitted, in an emission spectral band (λₑₘ, λ_{fluo}), by the object (10) under the effect of said excitation beam (12), the emission-image sensor being able to acquire an emission image (Iₑₘ, I_{fluo}) on the basis of the collected emission light; and
- a distance sensor (20) including a rangefinding light source (21) that is able to emit, in a rangefinding spectral band (λ_{d}), a rangefinding beam (22) that propagates toward the object (10),
- the distance sensor also including a rangefinding-light sensor (26) that is able to detect rangefinding light (24) reflected, in the rangefinding spectral band (λ_{d}), by the object (10),
the device being **characterized in that** it includes:
- a projecting element (5a, 5f) that is configured to project said excitation beam (12) and said rangefinding beam (22) toward the object, in one and the same projection solid angle (Ω),
- the distance sensor (20) being able to measure, on the basis of a duration between the emission of the rangefinding beam (22) by the rangefinding-light source and the detection, by the rangefinding-light sensor, of the rangefinding light (24) reflected by the object, a shortest distance (d), in the projection solid angle, between the projecting element (5a, 5f) and the object; and
- a modulator (18) that is configured to modulate an intensity of the excitation beam (12) depending on said distance (d) measured by the distance sensor (20).

2. Observing device according to Claim 1, including a light guide (2) extending between a proximal end (3) and a distal end (5), the light guide being able to transmit:
- the excitation beam (12) and the rangefinding beam (22) from the proximal end (3) to the distal end (5); and
- said emission light (14) and rangefinding light emitted by the object (24) from the distal end (5) to the proximal end (3),
the distal end (5) of said light guide (2) including said projecting element (5a, 5f).

3. Observing device according to Claim 2, wherein the light guide includes, at its distal end (5), an optical system (5a) that is able to collect the emission light (14) and rangefinding light (24) coming from the object (10), so as to direct said light toward the proximal end (3) of the light guide.

4. Device according to Claim 3, wherein said optical system (5a) is able to direct the excitation beam (12) and the rangefinding beam (22) toward the object (10), the optical system (5a) then forming the projecting element.

5. Device according to Claim 2 or Claim 3, wherein:
- the light guide (2) includes at least one transmitting optical fiber (4f) extending between the proximal end (3) and the distal end of the light guide (2) so as to guide the excitation beam and the rangefinding beam between the proximal end (3) and the distal end (5),
- the end of each transmitting optical fiber (4f) at the distal end (5) forming the projecting element (5f).

6. Device according to any one of Claims 2 to 5, wherein the light guide includes a spectral splitter (6) that is able to direct:
- the emission light (14) toward the emission-image sensor (16); and
- the rangefinding light (24) reflected by the object (10) toward the rangefinding-light sensor (26).

7. Device according to any one of Claims 2 to 6, including:
- a visible-light source (31) that is able to emit, in a visible spectral band (λᵥᵢₛ), a visible-light beam (32) toward the object (10); and
- a visible-image sensor (36) that is able to collect visible light (34) reflected by the object under the effect of illumination by the visible-light beam (32), the visible-image sensor being able to acquire a visible image (Iᵥᵢₛ) on the basis of the collected visible light;
and wherein the light guide (2) is able to transmit:
- the visible-light beam (32) from the proximal end (3) to the distal end (5);
- and the visible light (34) reflected by the object (10) from the distal end (5) to the proximal end (3).

8. Device according to any one of the preceding claims, wherein the rangefinding-light source (21) and the excitation-light source (11) are one and the same.

9. Device according to any one of the preceding claims, wherein the distance sensor (20) includes:
- a distributor (28) that is able to redirect a portion of the rangefinding beam (22) emitted by the rangefinding source (22) toward a triggering photodetector (23), the latter being able to detect said rangefinding-beam portion redirected by said distributor (28); and
- a rangefinding processor (51) that is able to determine a distance (D) travelled by the rangefinding beam (22) between the rangefinding-light source (21) and the rangefinding sensor (26) on the basis of a trigger time at which the triggering photodetector (23) detects the rangefinding beam (22) and of an end time at which the rangefinding-light sensor (26) detects the rangefinding light reflected by the object,
- the rangefinding processor (51) being able to determine the distance (d) between the projecting element (5a, 5f) and the object (10) on the basis of the distance (D) travelled by the rangefinding beam.

10. Method for observing an object, including the following steps:
- illuminating the object (10) with a rangefinding-light beam (22) emitted, in a rangefinding spectral band (λ_{d}), by a rangefinding-light source (21), the rangefinding beam being projected onto the object by a projecting element (5a, 5f) in a projection solid angle (Ω);
- detecting rangefinding light (24) reflected, in said rangefinding spectral band (λ_{d}), by the object;
- measuring a distance (d) between the projecting element (5a, 5f) and the object (10) on the basis of a duration between the emission of the rangefinding beam (22) and the detection of the rangefinding light (24) reflected by the object;
- illuminating said object (10) using an excitation beam (12) emitted, in an excitation spectral band (λₑₓ), by an excitation-light source (11), the excitation beam being projected onto the object by the projecting element (5a, 5f) in the projection solid angle (Ω);
- detecting emission light (14) with an emission-image sensor (16), the emission light (14) being emitted by the object (10) under the effect of the illumination by the excitation beam (12); and
- acquiring an emission image (Iₑₘ, I_{fluo}) with said emission-image sensor (16) on the basis of said detected emission light (14),
the method being **characterized in that** it also includes:
- modulating an intensity of the excitation beam (12) depending on the measured distance (d), the measured distance corresponding to the shortest distance, in the projection solid angle (Ω), between the projecting element (5a, 5f) and the object.

11. Method according to Claim 10, wherein:
- the excitation beam (12) and the rangefinding-light beam (22) are transmitted to the object (10) by a light guide (2) a distal end (5) of which, comprising the projecting element (5a, 5f), is placed facing the object (10),
- the light guide also ensuring transmission of the emission light (14) emitted by the object and the rangefinding light (24) reflected by the object to the emission-image sensor (16) and the rangefinding-light sensor (26), respectively.

12. Method according to Claim 11, wherein the emission light (14) emitted by the object and the rangefinding light (24) reflected by the object are collected by an optical system (5a) that is located at the distal end (5) of the light guide (2), before being redirected toward the emission-image sensor (16) and the rangefinding-light sensor (26).

13. Method according to Claim 12, wherein the excitation beam (12) and the rangefinding beam (14) are projected onto the object (10) by the optical system (5a), the latter forming said projecting element (5a).

14. Method according to Claim 11, wherein the excitation beam (12) and the rangefinding beam (14) are projected onto the object (10) by at least one optical fiber (4f) extending between the proximal end (3) and the distal end (5) of the light guide (2), the end of each optical fiber at said distal end (5) forming the projecting element (5f).

15. Method according to any one of Claims 10 to 14, wherein the emission light is fluorescence light in a fluorescence spectral band (λ_{fluo}) that is different from the excitation spectral band (λₑₓ).

16. Method according to any one of Claims 10 to 15, also including:
- illuminating, through the light guide (2), the object with a visible-light beam (32) produced, in a visible spectral band (λᵥᵢₛ), by a visible-light source (31); and
- acquiring a visible image (Iᵥᵢₛ) of the object (10) using a visible-image sensor (36), the latter detecting visible light reflected by the object (34) through the light guide (2).
